# EUROPEAN PATENT APPLICATION

(11) **EP 2 325 185 A1**
(43) Date of publication of application: **25.05.2011**
(21) Application number: 09013587.2
(22) Date of filing: 28.10.2009
(51) Int. Cl.: C07D 475/04, A61K 31/519, A61P 35/00

(54) **Plk inhibitor**

(71) Applicant: GPC Biotech AG, 82152 Planegg-Martinsried (DE)
(72) Inventor: Brandstetter, Tilmann, Dr., 82061 Neuried (DE); Eickhoff, Jan, Dr., 80339 München (DE); Köstler, Roland, Dr., 82152 Martinsried (DE); Schoop, Andreas, Dr., 82061 Neuried (DE); Sennhenn, Peter, Dr., 80801 München (DE); Becker, Frank, Dr., 82152 Planegg (DE); Kauffmann, Christine, 81377 München (DE)
(74) Representative: Virnekäs, Bernhard

(57) **Abstract**

The present invention provides a derivative of dihydropteridinone. This compound is an inhibitor of Polo-like kinases (Plks), that shows anti-proliferative activity against cells, including against tumor cells, and is useful in the treatment of diseases including cancer.

## Description

### Field of the Invention

The present invention provides a derivative of dihydropteridinone. This compound is an inhibitor of Polo-like kinases (Plks), that shows anti-proliferative activity against cells, including against tumor cells, and is useful in the treatment of diseases including cancer.

### Background of the Invention

Kinases are important cellular enzymes that perform essential cellular functions such as regulating cell division and proliferation, and appear to play a decisive role in many disease states such as in disease states that are characterized by uncontrolled proliferation and differentiation of cells. These disease states encompass a variety of cell types and maladies such as cancer, atherosclerosis, and restenosis.

Drugs that target kinases, and especially drugs based on small-molecule chemical compounds that inhibit the activity of particular kinases, are important medicines that can significantly improve the health, lifespan or quality of life for people throughout the world, including patients suffering from highly debilitating diseases such as cancer. Indeed, the drug "imatinib" (GLEEVEC/GLIVEC; Novartis) is such a drug that selectively inhibits one kind of kinase and is used to successfully treat certain types of cancer including particular types of leukaemia (CML) and gastrointestinal cancer (GIST). Its improved efficacy compared to previous standard treatments for CML, and that it is generally very well tolerated, showing mild side-effects, has lead, among other reasons, for it to have made the cover of TIME magazine where it was described as a "bullet" to combat cancer.

Polo-like kinases (Plks) are important regulators of cell cycle progression during the M-phase. Named after the polo gene of Drosophila melanogaster, Plks are involved in the assembly and dynamics of the mitotic spindle apparatus and in the activation and inactivation of CDK/cyclin complexes.

Plk1 is a 601 amino acid protein that comprises an N-terminal kinase domain and a C-terminal regulatory domain that contains a sequence comprising 30 amino acid residues that is termed the Polo box. The motif is only observed in the Polo-like kinases. Its function remains unknown. It may be involved in an auto-regulatory mechanism or in targeting the kinase to substrates. Deletion of the C-terminal regulatory domain results in activation of the kinase. Plks have a threonine in the activation segment whose phosphorylation is essential for activity.

Plkl is principally expressed in G2 and M phases. It localizes at centrosomes in early mitosis, redistributes to the spindle and mitotic bridges at anaphase and is rapidly degraded by the APC/C complex during exit from mitosis. The numerous functions of Plkl at mitosis are well accounted for by the variety of substrates identified for this kinase, including nucleophosmin (Zhang et al.. (2004) J. Biol. Chem. 279, 35726-35734), Cdc25C phosphatase, cyclin B, a cohesin subunit of the mitotic spindle, subunits of the anaphase promoting complex, and mammalian kinesin-like protein 1 (MKLP-1) and other kinesin related motor proteins (Nigg et al. (1996) Exp Cell Res. 229,174-80). In addition to the regulation of mechanistic aspects of mitosis, Plkl contributes to set the timing of entry and transition through mitosis by phosphorylating APC/C subunits. Plkl has been associated with the regulation of the mitotic spindle checkpoint. In addition to its well described roles in mitosis, Plkl expression in NIH3T3 cells causes the formation of oncogenic foci. Such transformed cells are able to form tumors in nude mice.

Deregulation of Plk1 activity leads to the formation of aberrant centrosomes and mitotic spindle poles. Its overexpression is tightly correlated with the development of chromosomal instability and aneuploidy observed in neoplasias.

Upon selective inhibition of Plk1, mitotic progression is inhibited. Down-regulation of Plkl by antisense oligonucleotides and siRNA is highly effective in inhibiting cancer-cell proliferation both in vitro and in vivo (Spankuch-Schmitt B. et al (2002) J. Natl. Cancer Inst. 94, 1863-1877; Elez R. et al (2003) Oncogene 22, 69-80). Mitotically arrested cells displayed randomly separated condensed chromosomes and the occurrence of multiple spindle poles with well-formed asters (Schmidt et al (2006) Mol. Cancer Ther. 5, 809-817).

Two additional polo-like kinases with a Polo box have been identified in mammals, Plk2 (Snk) and Plk3 (Prk and Fnk). However, their roles are much less characterized. Plk2 has been shown to act during the G1 / S transition and to be involved in centriole duplication (Warnke et al. (2004) Curr. Biol. 14, 1200-1207). Plk3 has been shown to be required for entry into S phase (Zimmerman and Erikson (2007) Proc. Natl. Acad. Sci. USA 104, 1847-1852). Furthermore, it has been postulated that Plk3 exerts a tumor-suppressive activity (Xie et al. (2001) J. Biol. Chem. 276, 43305-12; Bahassi et al. (2002) Oncogene 21, 6633-40). Additionally, it was shown that Plkl and Plk3 act synergistically in cell cycle control (Ouyang et al. (1997) J. Biol. Chem. 272; 28646-51). Thus, since the functions of the various members of the Polo-like kinase family are only beginning to be understood in detail, kinase inhibitors that are specific for one or more of the Plks might be advantageous to mitigate off-target effects and hence potentially to diminish drug side-effects, and in any case would prove to be valuable tools useful to further elucidate the biology of the Polo-like kinases.

Despite the fact that the striking biological validation and the potential of Plk1 inhibitors in anticancer therapy has been assumed and described for almost ten years, there has been little progress in the discovery and development of small molecule Plkl inhibitors. Apart from the compound known as "BI 2536" (see below), there are no Plkl inhibitors in clinical trials to date. Just recently, the number of preclinical reports on small molecule Plkl inhibitors increased (McInnes C. et al. (2005) Curr. Topics Med. Chem. 5, 181-197; McInnes C. et al (2006) Nat. Chem. Biol. 608-17 (Epub 2006 Oct 8); Peters U. et al (2006) Nat. Chem. Biol. 618-26 (Epub 2006 Oct 8)).

In vitro and in vivo characterizations of BI 2536 demonstrated specific tumor killing activity of this clinical compound that inhibits Plkl with high potency (IC₅₀ = 0.83 nM). The compound showed more than 1,000-fold selectivity against a large panel of protein kinases, and also affected the activities of other Polo-like kinase family members, such as Plk2 (IC₅₀ = 3.5 nM) and Plk3 (IC₅₀ = 9 nM). Furthermore, BI 2536 was found to preferentially inhibit cycling cells over resting cells (Steegmaier et al. (2007) Curr. Biol. 17, 316-322; Lénárt et al (2007) Curr. Biol. 17, 304-315).

Thus, despite the progress that has been made, the search continues for low molecular weight kinase inhibitor compounds, especially compounds that inhibit Polo-like kinases such as Plk1, that are useful for treating a wide variety of diseases, including cancer, tumors and other proliferative disorders or diseases including restenosis, angiogenesis, diabetic retinopathy, psoriasis, surgical adhesions, macular degeneration, and atherosclerosis, or other disorders or diseases mentioned herein. Compounds that inhibit a particular kinase or family of kinases, such as Polo-like kinases, without strongly inhibiting other kinases, are particularly sought after as they are presumed to show reduced off-target effects and hence predicted to cause milder side-effects when used as a drug. Thus, a strong need exists to provide compositions, pharmaceuticals and/or medicaments with kinase inhibitory activity, particularly with inhibitory activity against one or more members of the Polo-like kinases such as Plk1, or with anti-proliferative activity against cells such as tumour cells. Particularly, such compositions, pharmaceuticals and/or medicaments that have selective inhibitory activity against certain kinases or a kinase-family such as the Plks, may possess not only such activity, may also exert tolerable, acceptable, milder or diminished side-effects in comparison to other anti-proliferative or anti-cancer agents. Furthermore, the spectrum of tumors or other diseases responsive to treatment with such compositions, pharmaceuticals and/or medicaments may be broad. The active ingredients of such compositions, pharmaceuticals and/or medicaments may be suitable in the mentioned indication as single agent, and/or in combination therapy, be it in connection with other therapeutic agents, with radiation, with operative/surgical procedures, heat treatment or any other treatment known in the mentioned indications.

The potential significance of the Polo-like kinases in the cell-proliferative process, and hence the expected utility of the Plks as drug targets for anti-proliferative agents, thus generates a need to fmd compounds that are useful to help further elucidate the role and function of the various members of the Plk family. New small-molecule compounds that selective inhibit the Plks compared to other kinases, or in particular show selective inhibition of Plk1 activity, will be valuable tools to use within molecular- and cell-biological research when applied to the understanding of the Polo-like kinases and hence can provide important assistance in the identification of other compounds that could be developed and used as drugs that target Plks, including Plk1.

It is known that specific classes of dihydropteridines, substituted in a specific manner, have pharmacologically useful properties. In particular, specific derivatives of dihydropteridin-6-one, such as BI 2536, are known to possess anti-proliferative activity. These compounds however are structurally dissimilar from the compound of the present invention.

WO 03/020722 discloses substituted 2-(hetero)arylamino dihydropteridin-6-ones that are shown to be cytotoxic on HeLaS3 cells. Similarly, WO 2004/076454 discloses substituted 2-(4-carboxamidophenylamino)-5-methyl-dihydropteridin-6-ones, including BI 2536.

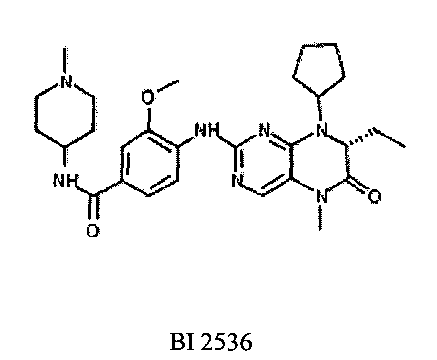

WO 2005/123736 discloses substituted 2-(hetero)arylinethylamino dihydropteridin-6-ones. WO 2006/021378 and WO 2006/021379 disclose substituted 2-(4-acylaminophenylamino)-5-methyl-dihydropteridin-6-ones, including the following structure:

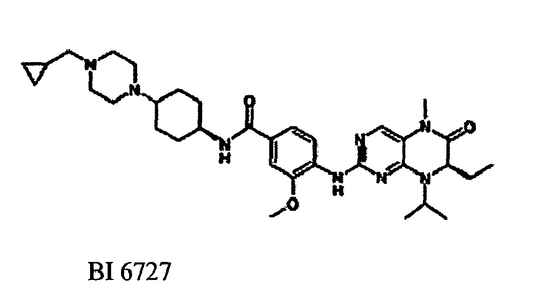

WO 2006/021548 discloses substituted 2-(4-aminophenylamino)-5-methyl-dihydropteridin-6-ones and 2-(4-aminopyridylamino)-5-methyl-dihydropteridin-6-ones. WO 2008/009909 discloses substituted 2-(hetero)arylamino-dihydropteridin-6-ones carrying an amino substituent at the nitrogen in position 5. In all these applications, the substituent at the nitrogen at position 8 is either hydrogen or a C-linked substituent, as with BI 2536.

### Summary of the Invention

We have invented a derivative of 8-substituted dihydropteridin-6-ones that exhibits surprising properties, including potent and selective activity as inhibitors of Polo-like kinases, in particular potent inhibition of Plk1, or anti-proliferative activity against cells such as tumour cells. In particular, the compound exhibits reduced toxicity in animal experiments when compared to an analogue with different substitution at the nitrogen at position 8. Such derivative of dihydropteridin-6-one provides an opportunity to develop new and effective therapies for diseases associated with kinase de-regulation, especially de-regulation of Polo-like kinases, or cellular proliferation, such as cancer or inflammatory disorders. The selective inhibition of the activity of one or more Polo-like kinases compared to kinase from other families, and the reduced toxicity in animals, may lead to the development and use of the compound of the invention as drug with less off-target effects, and potentially showing milder or diminished treatment side-effects compared to alternative therapies. Furthermore, the compound of the invention being a selective inhibitor of one or more Polo-like kinases, can be used as valuable tool within molecular- and cellular-biological research to better understand the role and function of Plks, which in turn will be of importance in the identification of additional compounds that inhibit this important class of drug-targets.

The compound of the present invention is a specific derivative of dihydropteridin-6-one, as discussed in greater detail below. In analogy to certain dihydropteridin-6-ones previously described, the compound of the present invention is suitable for further pre-clinical or clinical research and development towards its use as drug for the treatment of a variety of disorders and diseases including cancer, proliferative, degenerative, inflammatory and other disorders and diseases. The invention, useful as either a drug-candidate or as a tool in research into Polo-like kinases, can lead to effective therapies and therapeutics for particularly debilitating diseases such as cancer and other diseases and disorders including those listed herein.

One aspect of the invention relates to the 8-substituted dihydropteridin-6-one having the structure represented by Formula (I) presented below, or tautomeric or stereoisomeric forms thereof, which is useful as kinase inhibitor, especially as inhibitor of one or more Polo-like kinases such as Plkl, and thus useful for treating proliferative disorders or diseases such as cancer, atherosclerosis, and restenosis, among others, or are useful as selective Plk inhibitors to further elucidate the function of Polo-like kinases

In another aspect, the invention relates to pharmaceutical compositions, including a pharmaceutically acceptable diluent, excipient or carrier and an amount, such as a therapeutically effective amount, of such kinase inhibitor, e.g., which is expected to ameliorate the effects of proliferative disorders or diseases including those mentioned herein, such as cancer.

Another aspect of the invention relates to a pharmaceutical package, including such pharmaceutical composition, and instructions which indicate that said pharmaceutical composition may be used for the treatment of a patient suffering from a proliferative disorder or disease, including those mentioned herein, such as cancer.

In another aspect, the invention relates to methods that involve administering to or contacting a subject, a cell, a tissue, an organ or an organism with a therapeutically effective amount of the compound or pharmaceutical composition disclosed herein. These methods include, but are not limited to, prophylaxis and/or treatment of a proliferative disorder or disease including those mentioned herein, such as cancer.

In another aspect, the invention relates to uses of the compound of the present invention for the preparation of a medicament for the treatment of a proliferative disorder or disease, including those mentioned herein, such as cancer.

Another aspect of the invention related to the use of the compound of the present invention for the preparation of a composition for the inhibition of one or more Polo-like kinase, and the use of such compound or composition to inhibit such kinase, including the inhibition of Plk1.

Another aspect of the invention relates to methods of synthesizing the compound of the present invention, and to intermediates for such compound.

Accordingly, the present invention provides the compound having the structure represented by Formula (I) 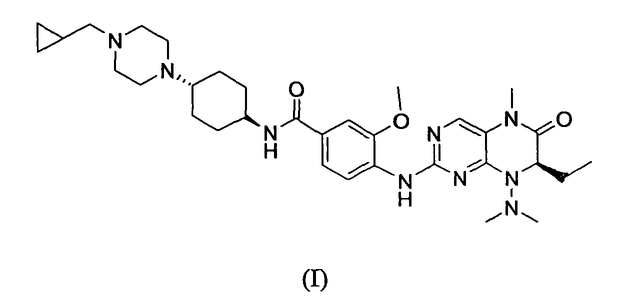 or any tautomeric or stereoisomeric form thereof, provided that the stereoisomeric forms specifically designated in Formula (I), wherein the ring carbon atom in position 7 of the pteridinone ring system has the (R)-configuration, and the 1,4-substitution of the cyclohexane ring is in trans-configuration, are not changed and kept in the (R)-, and trans-configuration, respectively;
or any pharmaceutically acceptable salt or N-oxide thereof.

The compound of the structure is shown in Table 1 below. In case of a discrepancy between the chemical name and the structure shown in Table 1, the structure should be regarded as correct, and the name amended accordingly.

Other features and advantages of the invention will be apparent from the following detailed description and from the claims.

### Detailed Description of the Invention

### Definitions

The term "tautomeric or stereoisomeric form thereof, provided that the stereoisomeric forms specifically designated in formula (I) [...] are not changed" as used herein includes all possible stereoisomeric and tautomeric forms of the compound of the present invention, provided that the stereoisomeric forms at the ring carbon atom in position 7 of the pteridinone ring system, which is in the (R)-configuration, and of the 1,4-substitution of the cyclohexane ring, which is in trans-configuration, and not changed and kept in the (R)-, and trans-configuration, respectively.

The present invention is intended to include all isotopes of atoms occurring on the present compound. Isotopes are atoms having the same atomic number but different mass numbers. By way of general example and without limitation, isotopes of hydrogen include tritium and deuterium. Isotopes of carbon include ¹²C and ¹⁴C.

The term "metabolite", as used herein, refers to any substance produced by the metabolism or by a metabolic process. Metabolism, as used herein, refers to the various physical/chemical/biochemical/pharmacological reactions involved in the transformation of molecules or chemical compounds occurring in the cell, tissue, system, body, animal, individual, patient or human therein.

The term "IC₅₀", as used herein, refers to concentrations at which a measurable activity, phenotype or response, for example growth or proliferation of cells such as tumor cells, is inhibited by 50%. IC₅₀ values can be estimated from an appropriate dose-response curve, for example by eye or by using appropriate curve fitting or statistical software. More accurately, IC₅₀ values may be determined using non-linear regression analysis.

As used herein, an "individual" means a multi-cellular organism, for example an animal such as a mammal, including a primate. In addition to primates, such as humans, a variety of other mammals can be treated according to a method that utilizes the compound of the present invention. For example, mammals including, but not limited to, cows, sheep, goats, horses, dogs, cats, guinea pigs, rats or other bovine, ovine, equine, canine, feline, rodent, or murine species can be used.

As used herein, a "proliferative disorder" or a "proliferative disease" includes a disease or disorder that affects a cellular growth, differentiation, or proliferation process.

As used herein, a "cellular growth, differentiation or proliferation process" is a process by which a cell increases in number, size or content, by which a cell develops a specialized set of characteristics which differ from that of other cells, or by which a cell moves closer to or further from a particular location or stimulus. A cellular growth, differentiation, or proliferation process includes amino acid transport and degradation and other metabolic processes of a cell. A cellular proliferation disorder may be characterized by aberrantly regulated cellular growth, proliferation, differentiation, or migration. Cellular proliferation disorders include tumorigenic diseases or disorders.

As used herein, a "tumorigenic disease or disorder" includes a disease or disorder characterized by aberrantly regulated cellular growth, proliferation, differentiation, adhesion, or migration, which may result in the production of or tendency to produce tumors. As used herein, a "tumor" includes a benign or malignant mass of tissue. Examples of cellular growth or proliferation disorders include, but are not limited to tumors, cancer, autoimmune diseases, viral diseases, fungal diseases, neurodegenerative disorders and cardiovascular diseases.

As used herein, the terms "anti-cancer agent" or "anti-proliferative agent" refer to compounds with anti-cancer and anti-proliferative properties, respectively. These compounds include, but are not limited to, altretamine, busulfan, chlorambucil, cyclophosphamide, ifosfamide, mechlorethamine, melphalan, thiotepa, cladribine, fluorouracil, floxuridine, gemcitabine, thioguanine, pentostatin, methotrexate, 6-mercaptopurine, cytarabine, carmustine, lomustine, streptozotocin, carboplatin, cisplatin, oxaliplatin, picoplatin, LA-12, iproplatin, tetraplatin, lobaplatin, JM216, JM335, satraplatin, fludarabine, aminoglutethimide, flutamide, goserelin, leuprolide, megestrol acetate, cyproterone acetate, tamoxifen, anastrozole, bicalutamide, dexamethasone, diethylstilbestrol, prednisone, bleomycin, dactinomycin, daunorubicin, doxirubicin, idarubicin, mitoxantrone, losoxantrone, mitomycin-c, plicamycin, paclitaxel, docetaxel, topotecan, irinotecan, 9-amino camptothecan, 9-nitro camptothecan, GS-211, JM 118, etoposide, teniposide, vinblastine, vincristine, vinorelbine, procarbazine, asparaginase, pegaspargase, octreotide, estramustine, and hydroxyurea. Said terms also include, but are not limited to, non-small molecule therapeutics, such as antibodies, e.g., 1D09C3 and other anti-HLA-DR antibodies as described in WO 01/87337 and WO 01/97338, Rituxan as described in US patents 5,736,137, 5,776,456, 5,843,437, 4D5, Mab225, C225, Daclizumab (Zenapax), Antegren, CDP 870, CMB-401, MDX-33, MDX-220, MDX-477, CEA-CIDE, AHM, Vitaxin, 3622W94, Therex, 5G1.1, IDEC-131, HU-901, Mylotarg, Zamyl (SMART M195), MDX-210, Humicade, LymphoCIDE, ABX-EGF, 17-1A, Trastuzumab (Herceptin ®, rhuMAb), Epratuzumab, Cetuximab (Erbitux ®), Pertuzumab (Omnitarg®, 2C4), R3, CDP860, Bevacizumab (Avastin ®), tositumomab (Bexxar ®), Ibritumomab tiuxetan (Zevalin ®), M195, 1D10, Hu1D10 (Remitogen®, apolizumab), Danton/DN1924, an "HD" antibody such as HD4 or HD8, CAMPATH-1 and CAMPATH-1H or other variants, fragments, conjugates, derivatives and modifications thereof, or other equivalent compositions with improved or optimized properties, and proteins or peptides, e.g., those described in Trends in Biotechnology (2003), 21(12), p.556-562.

As used herein, an "inflammatory disorder" or an "inflammatory disease" includes a disease or disorder that is caused or accompanied by inflammatory processes. This includes, but is not limited to, diseases or disorders such as arthritis, including but not limited to rheumatoid arthritis, spondyloarthropathies, gouty arthritis, osteoarthritis, systemic lupus erythematosus and juvenile arthritis, osteoarthritis, gouty arthritis and other arthritic conditions; pulmonary disorders or lung inflammation, including adult respiratory distress syndrome, pulmonary sarcoidosis, asthma, silicosis, and chronic pulmonary inflammatory disease; viral and bacterial infections, including sepsis, septic shock, gram negative sepsis, malaria, meningitis, cachexia secondary to infection or malignancy, cachexia secondary to acquired immune deficiency syndrome (AIDS), AIDS, ARC (AIDS related complex), pneumonia, and herpes virus; bone resorption diseases, such as osteoporosis, endotoxic shock, toxic shock syndrome, reperfusion injury, autoimmune disease including graft vs. host reaction and allograft rejections, cardiovascular diseases including atherosclerosis, thrombosis, congestive heart failure, and cardiac reperfusion injury, renal reperfusion injury, liver disease and nephritis, and myalgias due to infection; Alzheimer's disease, influenza, multiple sclerosis, cancer, diabetes, systemic lupus erythematosus (SLE), skin-related conditions such as psoriasis, eczema, bums, dermatitis, keloid formation, and scar tissue formation; gastrointestinal conditions such as inflammatory bowel disease, Crohn's disease, gastritis, irritable bowel syndrome and ulcerative colitis; ophthalmic diseases, such as retinitis, retinopathies, uveitis, ocular photophobia, and of acute injury to the eye tissue; angiogenesis, including neoplasia; metastasis; ophthalmological conditions such as corneal graft rejection, ocular neovascularization, retinal neovascularization including neovascularization following injury or infection, diabetic retinopathy, retrolental fibroplasia and neovascular glaucoma; ulcerative diseases such as gastric ulcer; pathological, but non-malignant, conditions such as hemangiomas, including infantile hemangiomas, angiofibroma of the nasopharynx and avascular necrosis of bone; diabetic nephropathy and cardiomyopathy; and disorders of the female reproductive system such as endometriosis.

As used herein, "pharmaceutically acceptable salts" refers to derivatives of the disclosed compound wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, palmoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from a parent compound which contains a basic or acidic moiety by conventional chemical methods. Generally, such salts can be prepared by reacting the free acid or base forms of the compound with a stoichiometric amount of the appropriate base or acid in water or in an organic solvent, or in a mixture of the two; generally, nonaqueous media like ether, EtOAc, ethanol, isopropanol, or acetonitrile are preferred. Lists of suitable salts are found in Remington's Pharmaceutical Sciences, 18th ed., Mack Publishing Company, Easton, PA, 1990, p. 1445, the disclosure of which is hereby incorporated by reference.

Any salt that retains the desired biological activity of the compound contained herein and that exhibits minimal or no undesired or toxicological effects is intended for inclusion here. Pharmaceutically acceptable salts include those derived from pharmaceutically acceptable organic or inorganic acids and bases. Non-pharmaceutically acceptable acids and bases also find use herein, as for example, in the synthesis and/or purification of the compound of interest. Thus, all "salts" are also encompassed within the scope of the instant invention.

Non-limiting examples of suitable salts include those derived from inorganic acids, such as, for example, hydrochloric acid, hydrobromic acid, sulfuric acid, phosphoric acid, nitric acid, bicarbonic acid, carbonic acid; and salts formed with organic acids, such as, for example, formic acid, acetic acid, oxalic acid, tartaric acid, succinic acid, malic acid, malonic acid, ascorbic acid, citric acid, benzoic acid, tannic acid, palmoic acid, alginic acid, polyglutamic acid, tosic acid, methanesulfonic acid, naphthalenesulfonic acid, naphthalenedisulfonic acid, α-ketoglutaric acid, β-glycerophosphoric acid and polygalacturonic acid. Suitable salts include those derived from alkali metals such as lithium, potassium and sodium, from alkaline earth metals such as calcium and magnesium, as well as from other acids well known to those of skill in the pharmaceutical art. Other suitable salts include those derived from metal cations such as zinc, bismuth, barium, or aluminum, or with a cation formed from an amine, such as ammonia, N,N-dibenzylethylenediamine, D-glucosamine, tetraethylammonium, or ethylenediamine. Moreover, suitable salts include those derived from a combination of acids and bases, such as, for example, a zinc tannate salt.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The term "prodrug", as used herein, refers to an agent that is converted into a pharmacologically active parent drug in vivo, such as the compound as defmed herein. The term "prodrug" includes any covalently bonded carriers that release an active parent drug of the present invention in vivo when such prodrug is administered to an animal. Since prodrugs are known to enhance numerous desirable qualities of pharmaceuticals (e.g., solubility, bioavailability, manufacturing, transport, pharmacodynamics, etc.), the compound of the present invention may be delivered in prodrug form. Prodrugs, for instance, may be bioavailable by oral administration even when the parent drug is not. Thus, the present invention is intended to cover prodrugs of the presently disclosed compound, methods of delivering the same, and compositions containing the same. Prodrugs of the present invention are prepared by modifying functional groups present in the compound in such a way that the modifications are cleaved, either in routine manipulation or in vivo, to the parent compound. Prodrugs include a compound of the present invention, wherein an amino group is bonded to any group that, when the prodrug of the present invention is administered to a mammalian subject, it cleaves to form the free amino group. Examples of prodrugs include, but are not limited to, acetate, formate, and benzoate derivatives of the amine functional groups in the compound of the present invention.

Generally speaking, prodrugs are derivatives of *per se* drugs that after administration undergo conversion or metabolism to the physiologically active species. The conversion may be spontaneous, such as hydrolysis in the physiological environment, or may be enzyme-catalyzed. Prodrugs include compounds that can be oxidized, reduced, aminated, deaminated, hydroxylated, dehydroxylated, hydrolyzed, esterified, alkylated, dealkylated, acylated, deacylated, phosphorylated, and/or dephosphorylated to produce the active compound.

From among the voluminous scientific literature devoted to prodrugs in general, the foregoing examples are cited: Gangwar et al., "Prodrug, molecular structure and percutaneous delivery", Des. Biopharm. Prop. Prodrugs Analogs, [Symp.] Meeting Date 1976, 409-21. (1977); Nathwani and Wood, "Penicillins: a current review of their clinical pharmacology and therapeutic use", Drugs 45(6): 866-94 (1993); Sinhababu and Thakker, "Prodrugs of anticancer agents", Adv. Drug Delivery Rev. 19(2): 241-273 (1996); Stella et al., "Prodrugs. Do they have advantages in clinical practice?", Drugs 29(5): 455-73 (1985); Tan et al. "Development and optimization of anti-HIV nucleoside analogs and prodrugs: A review of their cellular pharmacology, structure-activity relationships and pharmacokinetics", Adv. Drug Delivery Rev. 39(1-3): 117-151 (1999); Design of Prodrugs (Bundgaard H. ed.) 1985 Elsevier Science Publishers B. V. (Biomedical Division), Chapter 1; Design of Prodrugs: Bioreversible derivatives for various functional groups and chemical entities (Hans Bundgaard); Bundgaard et al. Int. J. of Pharmaceutics 22 (1984) 45 - 56 (Elsevier); Bundgaard et al. Int. J. of Pharmaceutics 29 (1986) 19 - 28 (Elsevier); Bundgaard et al. J. Med. Chem. 32 (1989) 2503 - 2507 Chem. Abstracts 93, 137935y *(Bundgaard et al.);* Chem. Abstracts 95, 138493f *(Bundgaard et al.*); Chem. Abstracts 95, 138592n *(Bundgaard et al.*); Chem. Abstracts 110, 57664p *(Alminger et al.*); Chem. Abstracts 115, 64029s *(Buur et al.*); Chem. Abstracts 115, 189582y (*Hansen et al.*); Chem. Abstracts 117, 14347q *(Bundgaard et al.*); Chem. Abstracts 117, 55790x *(Jensen et al.);* and Chem. Abstracts 123, 17593b *(Thomsen et al.).*

The terms "administered", "administration", or "administering" a compound will be understood to mean providing the compound of the invention to an individual, including an animal, in need of treatment by bringing such individual in contact with, or otherwise exposing such individual to, such compound.

The term "in vitro" refers to a biological entity, a biological process, or a biological reaction outside the body in artificial conditions. For example a cell grown in vitro is to be understood as a cell grown in an environment outside the body, e.g., in a test tube, a culture tray or a microtiter plate.

The term "therapeutically effective amount" means the amount of the subject compound that will elicit the biological, physiological, pharmacological, therapeutic or medical response of a cell, tissue, system, body, animal, individual, patient or human that is being sought by the researcher, scientist, pharmacologist, pharmacist, veterinarian, medical doctor, or other clinician, e.g., lessening of the effects/symptoms of a disorder or disease, such as a proliferative disorder or disease, for example, a cancer or tumor, or killing or inhibiting growth of a proliferating cell, such as a tumor cell. The therapeutically effective amount can be determined by standard procedures, including those described below in the section "Dosages".

The term "further treated", "further administer", or "further administered" means that different therapeutic agents or compounds may be administered together, alternatively or intermittently. Such further administration may be temporally or spatially separated, for example, at different times, on different days or via different modes or routes of administration.

In one embodiment of the present invention, the compound of the invention has a purity of more than 90%, more than 95%, more than 98%, or more than 99%. The compound may exist in one or more crystalline forms, including two or more polymorphic forms, and may exist as a dry solid or as a solvate including a defmed amounts of a solvent, including a hydrate including defmed amounts of water.

In another embodiment, the compound of the invention is the planned and deliberate product of a synthetic chemistry scheme, i.e., produced by specific and planned chemical processes conducted in reaction vessels, and not by degradation, metabolism or fermentation, or produced as impurity or by-product in the synthesis of other compounds.

In certain embodiments, the compound of the invention is purified or isolated, e.g., to have a purity of at least 80%, preferably at least 90%, more preferably at least 95%, such as at least 97%, at least 98% or even at least 99%. Purity, as used herein, can refer to either absolute or relative purity. Absolute purity refers to the amount of the compound of the invention obtained as the product of a synthetic chemistry scheme, either before or after one or more purification steps. Relative purity refers to the amount of the compound of the invention relative to one or more impurities such as by-products, degradation products (e.g., metabolites, products of oxidation or hydrolysis, etc.) and/or compounds that degrade to form the compound of the invention (e.g., precursors or prodrugs), e.g., that may be present in the product of a synthetic chemistry scheme. Thus, absolute purity refers to the amount of a compound relative to all others, while relative purity is generally unaffected by the addition of unrelated compounds, such as excipients, stabilizers, or other medicaments for conjoint administration. Purity can be assessed based upon weight, volume or molar ratios of one compound relative to others. Purity can be measured by a variety of analytical techniques, including elemental abundance, UV-visible spectrometry, HPLC, GC-MS, NMR, mass spectrometry, and thin layer chromatography, preferably by HPLC, GC-MS, or NMR.

Yet another aspect of the invention relates to prodrugs of the compound described above.

### Formulations, Dosages and Applications

The present invention further provides a pharmaceutical composition including the compound as described above, or prodrug thereof, and a pharmaceutically acceptable diluent, excipient or carrier, including pharmaceutical compositions including a therapeutically effective amount of such compound or prodrug.

### Formulations

The compound of this invention can be formulated and administered to treat individuals in need by any means that produces contact of the active ingredient with the agent's site of action, such as a cell, in the body of an individual. It can be administered by any conventional means available for use in conjunction with pharmaceuticals, either as individual therapeutic active ingredients or in a combination of therapeutic active ingredients. It can be administered alone, but are generally administered with a pharmaceutically acceptable diluent, excipient or carrier selected on the basis of the chosen route of administration and standard pharmaceutical practice.

A pharmaceutical composition comprising less than a therapeutically effective amount of the compound described above, or a prodrug thereof, may also be used, such as when used in combination with another pharmaceutical composition, such as an anti-cancer agent, so that such combination is therapeutically effective, or may be useful for prophylactic treatment.

Pharmaceutical compositions for use in accordance with the present invention may be formulated in conventional manner using one or more pharmaceutically acceptable diluents, excipients or carriers. The pharmaceutical compositions of the invention can be formulated for a variety of routes of administration, including systemic and topical or localized administration. Techniques and formulations generally may be found in Remington's Pharmaceutical Sciences, Meade Publishing Co., Easton, PA. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, capsules, boluses, powders, granules, pastes for application to the tongue; (2) parenteral administration, for example, by subcutaneous, intramuscular or intravenous injection as, for example, a sterile solution or suspension; (3) topical application, for example, as a cream, ointment or spray applied to the skin; or (4) intravaginally or intrarectally, for example, as a pessary, cream or foam. In certain embodiments, the pharmaceutical preparations may be non-pyrogenic, i.e., do not substantially elevate the body temperature of a patient.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of inhibitor which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association the compound of the present invention with the carrier and, optionally, one or more accessory ingredients. In general, the formulations are prepared by uniformly and intimately bringing into association the compound of the present invention with liquid carriers, or fmely divided solid carriers, or both, and then, if necessary, shaping the product.

For systemic administration, injection is preferred, including intramuscular, intravenous, intraperitoneal, and subcutaneous (i.m., i.v., i.p., and s.c. respectively). The phrases "systemic administration", "administered systemically", "peripheral administration", and "administered peripherally" as used herein mean the administration of a compound, drug or other material other than directly into the central nervous system, such that it enters the patient's system and, thus, is subject to metabolism and other like processes, for example, subcutaneous administration.

For injection, the pharmaceutical compositions of the invention can be formulated in liquid solutions, preferably in physiologically compatible buffers such as Hank's solution or Ringer's solution. In addition, the pharmaceutical compositions may be formulated in solid form and redissolved or suspended immediately prior to use. Lyophilized forms are also included.

Pharmaceutical compositions of the invention may be formulated to be suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of the compound of the present invention as an active ingredient. The compound of the present invention may also be administered as a bolus, electuary or paste.

In formulating the pharmaceutical compositions of the invention in solid dosage forms for oral (p.o.) administration (capsules, tablets, pills, dragees, powders, granules and the like), the compound of the invention as active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, high molecular weight polyethylene glycols, and the like.

Gelatin capsules contain the compound of the present invention as active ingredient and powdered carriers, such as lactose, starch, cellulose derivatives, magnesium stearate, stearic acid, and the like. Similar carriers can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric coated for selective disintegration in the gastrointestinal tract. Solid compositions of a similar type are also employed as fillers in soft and hard-filled gelatin capsules; preferred materials in this connection also include lactose or milk sugar as well as high molecular weight polyethylene glycols. A preferred formulation is a solution or suspension in an oil, for example olive oil, Miglyol, or Capmul, in a soft gelatin capsule. Antioxidants may be added to prevent long-term degradation as appropriate.

A tablet may be made by compression or molding, optionally with one or more accessory ingredients. Compressed tablets may be prepared using a binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered inhibitor moistened with an inert liquid diluent.

The tablets and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulations so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the pharmaceutical compositions of the invention include pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the pharmaceutical compositions for oral administration can also include adjuvants such as wetting agents, emulsifying and suspending agents, sweetening, flavoring, coloring, perfuming, and preservative agents.

Suspensions, in addition to the pharmaceutical composition of the present invention, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

For buccal administration the pharmaceutical compositions may take the form of tablets or lozenges formulated in a conventional manner.

For administration by inhalation, the pharmaceutical compositions of the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the therapeutic agents and a suitable powder base such as lactose or starch.

The pharmaceutical compositions may be formulated for parenteral administration by injection, e.g., by bolus injection or continuous infusion. Formulations for injection may be presented in unit dosage form, e.g., in ampoules or in multi-dose containers, with an added preservative. The pharmaceutical compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Pharmaceutical compositions of this invention suitable for parenteral administration comprise one or more inhibitors of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These pharmaceutical compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the pharmaceutical compositions. In addition, prolonged absorption of the injectable pharmaceutical form may be brought about by the inclusion of agents that delay absorption such as aluminum monostearate and/or gelatin.

In addition to the formulations described previously, the pharmaceutical compositions may also be formulated as a depot preparation. Such long acting formulations may be administered by implantation (for example subcutaneously or intramuscularly) or by intramuscular injection. Thus, for example, the pharmaceutical compositions may be formulated with suitable polymeric or hydrophobic materials (for example as an emulsion in an acceptable oil) or ion exchange resins, or as sparingly soluble derivatives, for example, as a sparingly soluble salt.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the pharmaceutical compositions of the invention are formulated into ointments, salves, gels, or creams as generally known in the art. A wash solution can be used locally to treat an injury or inflammation to accelerate healing.

In some cases, in order to prolong the therapeutic effect of an inhibitor, it is desirable to slow the absorption of the inhibitor from subcutaneous or intramuscular injection. This may be accomplished by the use of a liquid suspension of crystalline or amorphous material having poor water solubility. The rate of absorption of the inhibitor then depends upon its rate of dissolution which, in turn, may depend upon crystal size and crystalline form. Alternatively, delayed absorption of a parenterally administered inhibitor form is accomplished by dissolving or suspending the inhibitor in an oil vehicle.

Pharmaceutical compositions of the invention may be formulated for rectal or vaginal administration as a suppository, which may be prepared by mixing the compound of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active inhibitor.

Formulations of the pharmaceutical compositions of the present invention, which are suitable for vaginal administration, also include pessaries, tampons, creams, gels, pastes, foams or spray formulations containing such carriers as are known in the art to be appropriate.

Dosage forms for the topical or transdermal administration of the compound of this invention include powders, sprays, ointments, pastes, creams, lotions, gels, solutions, patches and inhalants. Such compound may be mixed under sterile conditions with a pharmaceutically acceptable carrier, and with any preservatives, buffers, or propellants which may be required.

The ointments, pastes, creams and gels may contain, in addition to the compound of the invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of the compound of the present invention to the body. Such dosage forms can be made by dissolving or dispersing an inhibitor of the present invention in the proper medium. Absorption enhancers can also be used to increase the flux of the drug across the skin. The rate of such flux can be controlled by either providing a rate controlling membrane or dispersing the compound of the present invention in a polymer matrix or gel.

Ophthalmic formulations, eye ointments, powders, solutions and the like, are also contemplated as being within the scope of this invention.

The pharmaceutical compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. In other embodiments, the pack or dispenser may be further packaged in an outer carton.

A pharmaceutical composition of the present invention can also be formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566, the disclosures of which are each incorporated herein by reference. The pharmaceutical compositions of the present invention can be used to provide slow or sustained release of one or more of the active ingredients using, for example, hydroxypropylmethyl cellulose, other polymer matrices, gels, permeable membranes, osmotic systems, multilayer coatings, microparticles, liposomes, microspheres, or the like, or a combination thereof to provide the desired release profile in varying proportions. Suitable sustained release formulations known to those of ordinary skill in the art, including those described herein, may be readily selected for use with the pharmaceutical compositions of the invention. Thus, single unit dosage forms suitable for oral administration, such as, but not limited to, tablets, capsules, gelcaps, caplets, powders, and the like, that are adapted for sustained release are encompassed by the present invention.

Injectable depot forms are made by forming microencapsuled matrices of the subject inhibitors in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

When the compound of the present invention are administered as pharmaceuticals, to individuals, such as humans and animals, they can be given per se or as a pharmaceutical composition containing, for example, 0.1 to 99.5% (in certain embodiments, 0.5 to 90%) of active ingredient in combination with a pharmaceutically acceptable carrier.

The present invention provides new methods of treating proliferative, degenerative and other disorders or diseases, including cancer, by administering an amount such as a therapeutically effective amount of the compound disclosed herein or a prodrug, tautomeric, pharmaceutically acceptable salt, N-oxide or stereoisomeric form thereof. The present invention further provides methods of treating proliferative, degenerative or other disorders or diseases, including cancer, by administering a therapeutically effective combination of at least the compound disclosed herein and another anti-cancer or anti-proliferative agent.

The compound of the present invention may be administered as a salt or prodrug that, upon administration to the individual, is capable of providing directly or indirectly the parent compound, such as the compound as defmed herein, or that exhibits activity itself. Nonlimiting examples include a pharmaceutically acceptable salt, alternatively referred to as a "physiologically acceptable salt". In addition, modifications made to the compound can affect its biological activity, in some cases increasing the activity over the parent compound. This activity can be assessed by preparing a salt or prodrug form of the compound, and testing its activity by using methods described herein or other methods known to those of skill in the art.

As will be apparent to a person skilled in the art, through the use of a prodrug of a given subject compound, an individual such as an animal administered or treated with such prodrug will be exposed to, and hence indirectly administered with, the subject compound. Such a procedure may expose those cells associated with a disease, such as a proliferative disease or disorder including cancer, to the subject compound.

All processes used to prepare the compound of the present invention and intermediates made therein are considered to be part of the present invention.

### Dosages

A dosage administered that will be a therapeutically effective amount of the compound sufficient, or reasonably expected by a health-care professional such as a physician, pharmacist or nurse, to result in amelioration of symptoms of, for example, the cancer or tumor will, of course, vary depending upon known factors such as the pharmacodynamic characteristics of the particular active ingredient and its mode and route of administration; age, sex, health and weight of the recipient; nature and extent of symptoms; kind of concurrent treatment, frequency of treatment and the effect desired.

The subject compound may also be administered in prophylactic treatment. If the compound is administered prior to clinical manifestation of the unwanted condition (e.g., disease or other unwanted state of the host animal) then the treatment is prophylactic (i.e., it protects the individual against initiating, developing or further developing the unwanted condition). The subject compound may also be administered to prevent a condition, disorder or diseases, such as cancer, or a syndrome complex, such as heart failure or any other medical condition. This includes administration of the compound the intent of which is to reduce the frequency of, or delay the onset of, symptoms of a medical condition in an individual relative to an individual which does not receive the compound. Thus, prevention of cancer includes, for example, reducing the number of detectable cancerous growths, tumors, or malignancies in a population of patients receiving a prophylactic treatment relative to an untreated control population, delaying the appearance of detectable cancerous growths in a treated population versus an untreated control population, and/or delaying disease progression and/or improving the quality of patient life, e.g., by a statistically and/or clinically significant amount.

Toxicity and therapeutic efficacy of pharmaceutical compositions of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapeutic agents that exhibit large therapeutic indices are useful for many circumstances. In certain circumstances, even therapeutic compositions that appear to exhibit debilitating or toxic side effects may be used, including circumstances where care is taken to design a delivery system that targets such therapeutic agents to the site of affected tissue in order to minimize potential damage to unaffected cells and, thereby, reduce or localize side effects.

The data obtained from cell culture assays and animal studies can be used in formulating a range of dosage for use in humans. The dosage lies preferably within a range of circulating concentrations that include the ED50 with little or no toxicity. The dosage may vary within this range depending upon the dosage form employed and the route of administration utilized. For any agents used in the method of the invention, the therapeutically effective dose can be estimated initially from cell culture assays. A dose may be formulated in animal models to achieve a circulating plasma concentration range that includes the IC₅₀ (i.e., the concentration of the test therapeutic agent which achieves a half-maximal inhibition of symptoms or inhibition of biochemical activity) as determined in cell culture. Such information can be used to more accurately determine useful doses in humans. Levels in plasma may be measured, for example, by high performance liquid chromatography.

It is understood that appropriate doses of therapeutic agents depends upon a number of factors known to those or ordinary skill in the art, e.g., a physician. The dose(s) of the subject compound will vary, for example, depending upon the identity, size, and condition of the subject or sample being treated, further depending upon the route by which the composition is to be administered, if applicable, and the effect which the practitioner desires the therapeutic to have upon the therapeutic target of targets, such as cells, nucleic acid or polypeptides, through with the disease causes, symptoms or effects are mediated.

Exemplary doses include milligram or microgram amounts of the compound of the present invention per kilogram of subject or sample weight, e.g., about 1 microgram per kilogram to about 500 milligrams per kilogram, about 100 micrograms per kilogram to about 50 milligrams per kilogram, or about 1 milligram per kilogram to about 5 milligrams per kilogram.

A person skilled in the art will appreciate that doses can also be calculated on a body surface basis. A person of 70 kg has an approximate body surface area of 1.8 square meter, and doses can be expressed as milligram or microgram amounts of the compound per body surface area of subject or sample, e.g. about 50 microgram per square meter to about 15 grams per square meter, about 5 milligrams per square meter to about 1.5 grams per square meter, or about 50 milligram per square meter to about 150 milligrams per square meter.

### Applications

The present invention further provides the compound as described above for therapy. In other aspects, the invention provides the compound of the present invention for prophylatic uses.

In certain embodiments, said therapy or prophylactic use is the treatment or prevention of a proliferative disorder or disease, such as a tumor or cancer. In certain embodiments, said treatment is the treatment of a cancer that can be treated by the inhibition of the activity of a protein kinase or mutant thereof, such as the inhibition of the activity ofPlkl.

Thus, the present invention additionally provides a method for treating an individual, such as a mammal, having a disease-state selected from the group of proliferative disorders or diseases, or inflammatory disorders or diseases, comprising administering to said individual a therapeutically effective amount of the compound, a prodrug, or a pharmaceutical composition of the invention as described above. In certain embodiments, said individual is a human. In certain embodiments, said proliferative disorder or disease is cancer. In certain embodiments, said treatment is the treatment of a cancer that can be treated by the inhibition of the activity of a protein kinase or mutant thereof, such as the inhibition of the activity of Plk1.

The present invention also provides a method for prophylactic treatment of an individual such as an animal, including a mammal, particularly a human, the intent of which is to reduce the frequency of, delay the onset of, or the symptoms of a medical condition, such as cancer, in a subject relative to a subject which does not receive the composition.

In a further aspect, the invention provides methods of treating or preventing an individual suffering from a disease, such as a mammal, including a domestic mammal, cat, dog, horse, sheep, cow, rodent, and human, comprising the step of exposing said individual to an amount, including a therapeutically effective amount, of the subject compound. In certain embodiments, the disease is a proliferative disorder or disease, such as a cancer or tumour. In yet another embodiment, cells associated with said proliferative disorder or disease, including tumour cells included in a cancer, are exposed to the subject compound. In certain embodiments, said compound, or a prodrug thereof, is administered to said individual. In certain embodiments, said treatment is the treatment of a cancer that can be treated by the inhibition of the activity of a protein kinase or mutant thereof, such as the inhibition of the activity of Plkl. In certain embodiments, the disease is an inflammatory disorder or disease. In yet another embodiment, cells associated with said inflammatory disorder or disease are exposed to the subject compound. In certain embodiments, said compound, or a prodrug thereof, is administered to said individual.

In a further aspect, the invention provides a method of killing or inhibiting proliferation or growth of a cell, comprising contacting the cell with the compound of the invention. In one embodiment, the cell is cultured in-vitro, while in an alternative embodiment the cell is present in an individual. In a particular embodiment the cell is a cancer cell, for example a cell from a tumour cell line or a cell included in a tumour, including cancer cells from a tumour that can be treated by the inhibition of the activity of a protein kinase or mutant thereof, such as the inhibition of the activity of Plk1.

Yet another aspect of the invention relates to the use of the compound as described above, or a prodrug thereof, for the preparation of a medicament for the treatment or prevention of a proliferative disorder or disease, including cancer, including cancers that can be treated by the inhibition of the activity of a protein kinase or mutant thereof, such as the inhibition of the activity of Plk1. Additionally, the invention relates to a pharmaceutical composition comprising the compound as described above, or a prodrug thereof, and a pharmaceutically acceptable diluent, excipient or carrier, for the treatment of a proliferative disorder or disease, including cancer, including cancers that can be treated by the inhibition of the activity of a protein kinase or mutant thereof, such as the inhibition of the activity of Plk1.

The subject compound is useful to treat various disorders or diseases, including proliferative disorders or diseases. The term "proliferative disorder or disease" is also art recognized and includes a disorder or disease affecting an individual, such as an animal, in a manner which is marked by aberrant, or otherwise unwanted, proliferation of a subset of cells of an individual. Cancer and tumors are proliferative disorders or diseases. Cells comprising or derived from a tumor will generally be understood to be a proliferating cell, typically a hyper-proliferating cell, and in other circumstances, a tumor cell may be dysplastic, or may have proliferated. In certain embodiments, said treatment is the treatment of a cancer that can be treated by the inhibition of the activity of a protein kinase or mutant thereof, such as the inhibition of the activity of Plk1.

It will be apparent to a person skilled in the art, on reading the disclosure of the instant invention, that the methods, pharmaceutical compositions and packaged pharmaceuticals comprising the subject compound will be useful for the treatment of other proliferative disorders or diseases, or for killing or inhibiting proliferating cells including tumor cells.

the compound of the present invention may be useful in the treatment of disease processes which feature abnormal cellular proliferation, such as hyperproliferative diseases, including cancer, benign prostate hyperplasia, familial adenomatosis polyposis, neurofibromatosis, psoriasis, fungal infections, endotoxic shock, hypertrophic scar formation, inflammatory bowel disease, transplant rejection, vascular smooth muscle cell proliferation associated with atherosclerosis, psoriasis, pulmonary fibrosis, arthritis, glomerulonephritis, restenosis following angioplasty or vascular surgery, and other post-surgical stenosis and restenosis. See, for example, U.S. Patent Nos. 6,114,365 and 6,107,305.

The compound disclosed herein is expected to be useful in the therapy of proliferative or hyperproliferative disorders or diseases such as cancer, autoimmune diseases, viral diseases, fungal diseases, neurodegenerative disorders and cardiovascular disease.

In certain embodiments, tumors may be solid tumors, which are cancer of body tissues other than blood, bone marrow, or the lymphatic system. In other embodiments, tumors may be hematological tumors, such as leukemia and lymphomas. Leukemia is a collective term for malignant diseases characterized by a proliferation of malignantly changed white blood cells. Diseases arising from lymphatic tissue are called lymphomas.

Solid tumors may be selected from: liver cancer, stomach cancer, colon cancer, breast cancer, pancreas cancer, prostate cancer, skin cancer, renal cancer, bone cancer, thyroid cancer, skin cancer, including squamous cell carcinoma, esophagus cancer, kidney cancer, bladder cancer, gall cancer, cervical cancer, ovarian cancer, lung cancer, bronchial, small and non-small-cell lung cancer, gastric, and head and neck cancer.

Hematological tumors may be leukemia, such as Acute Myelogenous Leukemia (AML), Acute Lymphoblastic Leukemia (ALL), Acute Lymphocytic Leukemia, Acute Leukemia, Acute Promyelocytic Leukemia, Chronic Granulocytic Leukemia (CGL), Chronic Leukemia, Chronic Lymphocytic Leukemia (CLL), Chronic Myelogenous Leukemia (CML), Chronic Myelomonocytic Leukemia, Common-type Acute Lymphoblastic Leukemia, Eosinophilic Leukemia, Erythroleukemia, Extranodal Lymphoma, Follicular Lymphoma, Hairy Cell Leukemia, Monocytic Leukemia, Prolymphocytic Leukemia.

Hematological tumors may also be lymphoma, such as B Cell Lymphomas, Burkitt Lymphoma, Cutaneous T Cell Lymphoma, High-Grade Lymphoma, Hodgkin's Lymphoma, Non-Hodgkin's Lymphoma, Low-grade Lymphoma, Lymphoblastic Lymphoma, Mantle Cell Lymphoma, Marginal Zone Lymphoma, Mucosa-Associated Lymphoid Tissue (MALT) Lymphomas, T Cell Lymphomas, peripheral T cell lymphoma, multiple myeloma, Essential Thrombocythemia, Hairy Cell Lymphoma, Extramedullary myeloma, Granulocytic Sarcomae.

Hematological tumors may also be tumors of myeloid lineage, including acute and chronic myelogenous leukemias, myelodysplastic syndrome, and promyelocytic leukaemia.

Tumors may also be of mesenchymal origin, such as fibrosarcoma and rhabdomyosarcoma. Furthermore, tumors may be tumors of the central and peripheral nervous system, such as astrocytoma, neuroblastoma, glioma, and schwannomas; and tumors may be other tumors, such as melanoma, seminoma, teratocarcinoma, osteosarcoma, xenoderoma pigmentosum, keratoctanthoma, thyroid follicular cancer, and Kaposi's sarcoma.

Tumors that are resistant or refractory to treatment with other anti-cancer or anti-proliferative agents may also benefit from treatment with the methods and pharmaceutical compositions of the present invention.

The compound disclosed herein may also be useful in the chemoprevention of cancer. Chemoprevention is defined as inhibiting the development of invasive cancer by either blocking the initiating mutagenic event or by blocking the progression of pre-malignant cells, such as by blocking growth of the tumor, that have already suffered an insult or inhibiting tumor relapse.

The compound disclosed herein may also be useful in inhibiting tumor angiogenesis and metastasis.

The compound of this invention may also be useful in combination (administered together or sequentially) with known anti-cancer treatments such as radiation therapy or with anti-cancer, anti-proliferative, cytostatic or cytotoxic agents. Other anti-cancer and anti-proliferative agents which may be used in combination with the compound of the present invention include those described herein. In combination treatment, the compound of the present invention may be further administered with any other anti-cancer and anti-proliferative agent disclosed herein.

If formulated as a fixed dose, such combination products employ the compound of this invention within the dosage range described herein and the other pharmaceutically active agent or treatment within its approved dosage range. For example, the cdc2 inhibitor olomucine has been found to act synergistically with known cytotoxic agents in inducing apoptosis (J. Cell Sci., 108, 2897 (1995)). The compound described herein may also be administered sequentially with known anti-cancer or anti-proliferative agents when a combination formulation is inappropriate. The invention is not limited in the sequence of administration; the compound described herein may be administered either prior to or after administration of the known anti-cancer or anti-proliferative agent. For example, the cytotoxic activity of the cyclin-dependent kinase inhibitor flavopiridol is affected by the sequence of administration with anticancer agents (Cancer Research, 57, 3375 (1997)).

### Further Aspects of the Invention

Another aspect the invention provides a pharmaceutical package, wherein said package includes the compound of the present invention. In certain embodiments, the package comprises instructions which indicate that said composition may be used for the treatment of an individual in need thereof, including a human. In certain other embodiments, the pharmaceutical package includes the compound of the present invention formulated together with another pharmaceutical ingredient such as an anti-cancer or anti-proliferative agent. In this case, the compound of the present invention and the other pharmaceutical ingredient may be formulated separately and in individual dosage amounts.

Other pharmaceutical ingredients that may be formulated together or separately with the compound of the present invention include but are not limited to other anti-cancer and anti-proliferative agents such as described above. In certain still further embodiments, the pharmaceutical package comprises instructions to treat a patient in need of such treatment. In yet another aspect the invention provides a pharmaceutical package for treating an individual suffering from a proliferative disorder or disease, such as a tumor or a cancer, wherein said package includes at least the compound of the present invention. In certain still further embodiments, the pharmaceutical package comprises instructions to treat the disorder.

As used herein the term "pharmaceutical package" or "pharmaceutical pack" refer to any packaging system for storing and dispensing individual doses of medication. Preferably the pharmaceutical package contains sufficient daily dosage units appropriate to the treatment period or in amounts which facilitate the patient's compliance with the regimen. In certain embodiments, the pharmaceutical pack comprises one or more vessels that include the active ingredient, e.g., the compound of the present invention. Such vessel can be a container such as a bottle, vial, syringe, or capsule, or may be a unit dosage form such as a pill. The active ingredient may be provided in the vessel in a pharmaceutically acceptable form or may be provided, for example, as a lyophilized powder. In further embodiments, the pharmaceutical pack may further include a solvent to prepare the active ingredient for administration. In certain embodiments, the active ingredient may be already provided in a delivery device, such as a syringe, or a suitable delivery device may be included in the pack. The pharmaceutical package may comprise pills, liquids, gels, tablets, dragees or the pharmaceutical preparation in any other suitable form. The package may contain any number of daily pharmaceutical dosage units. The package may be of any shape, and the unit dosage forms may be arranged in any pattern, such as circular, triangular, trapezoid, hexagonal or other patterns. One or more of the doses or subunits may be indicated, for example to aid the doctor, pharmacist or patient, by identifying such dose or subunits, such as by employing color-coding, labels, printing, embossing, scorings or patterns. The pharmaceutical package may also comprise instructions for the patient, the doctor, the pharmacist or any other related person.

Some embodiments comprise the administration of more than one active ingredient, including the compound as disclosed herein. Such administration may occur concurrently or sequentially. The active ingredients may be formulated together such that one administration delivers both components. Alternatively the active ingredients may be formulated separately. The pharmaceutical package may comprise the compound of the present invention and the other pharmaceutical ingredient in a single formulation, i.e., they are formulated together, or the compound of the present invention and the other pharmaceutical ingredient in individual formulations, i.e., they are formulated separately. Each formulation may comprise the compound of the present invention and the other pharmaceutical ingredient in individual dosage amounts (in approximately equal or unequal amounts). Administration of the compound of the present invention and the other pharmaceutical ingredient results in a concentration that results in a therapeutically effective amount of the combination.

As used herein, the term "instructions" means a product label and/or documents or other information describing relevant materials or methodologies pertaining to assembly, preparation or use of a kit or packaged pharmaceutical. These materials may include any combination of the following: background information, steps or procedures to follow, list of components, proposed dosages, warnings regarding possible side effects, instructions for administering the drug, technical support, and any other related documents. Instructions can be supplied in printed form, such as a package label or a package insert. Instructions for a packaged pharmaceutical or a pharmaceutical composition can be inserted in a delivery carton or finished package, e.g., as a package insert, and the text of such has been approved by a competent regulatory authority such as the Food and Drug Administration (FDA) of the United States. Alternatively or complementarily, instruction may also be stored in electronic form, e.g., on a computer-readable storage medium such as a computer-readable memory device, a centralized database, magnetic media such as hard disks, floppy disks, and magnetic tape; optical media such as compact discs, CD-ROMs and holographic devices; magneto-optical media such as floptical disks; and hardware devices that are specially configured to store and execute program code, such as application-specific integrated circuits (ASICs), programmable logic devices (PLDs) and ROM (read only memory) and RAM (random access memory) devices. Instructions may comprise a web address of an internet website from which more detailed instructions may be downloaded, or a recorded presentation. Instructions can contain one or multiple documents or future updates.

Another aspect of the invention provides the use of the compound of Formula (I) to inhibit one or more Polo-like kinases. In another embodiment of this aspect, the compound of Formula (I) is used for the preparation of a composition for the inhibition of one or more Polo-like kinases. In yet another embodiment, the invention provides methods to inhibit one or more Polo-like kinase. Each of such embodiments include those where the Polo-like kinase is Plk1.

Thus, in one aspect the invention relates to a pharmaceutical composition, including the compound of the present invention, and a pharmaceutically acceptable diluent, excipient or carrier.

In certain embodiments, such pharmaceutical composition comprise a therapeutically effective amount of said compound or prodrug.

In certain embodiments, such pharmaceutical composition is for the treatment of an individual in need thereof.

In certain embodiments of such pharmaceutical composition, said individual is a human.

In another aspect the invention relates to a pharmaceutical package, including a pharmaceutical composition of the present invention, and instructions which indicate that said pharmaceutical composition may be used for the treatment of an individual in need thereof.

In certain embodiments of such pharmaceutical package, said instructions indicate that said pharmaceutical composition may be used for the treatment of a human.

In certain embodiments of such pharmaceutical package, said instructions indicate that said pharmaceutical composition may be used for the treatment of an individual suffering from a proliferative disorder or disease.

In certain such embodiments, said individual is a human.

In another aspect the invention relates to a method for treating a proliferative disorder or disease in an individual, comprising administering a therapeutically effective amount of the compound or a pharmaceutical composition of the present invention.

In certain embodiments of such method, said individual is a mammal selected from: domestic mammal, cat, dog, horse, sheep, cow, rodent, and human.

In certain embodiments of such method, said mammal is a human.

In another aspect the invention relates to a method for treating a proliferative disorder or disease in an individual, comprising exposing cells included in said disorder or disease to the compound of the present invention.

In certain embodiments of such method, said compound, or a prodrug thereof, is administered to said individual.

In certain embodiments of such method, said individual is a mammal selected from: domestic mammal, cat, dog, horse, sheep, cow, rodent, and human.

In certain embodiments of such method, said mammal is a human.

In another aspect the invention relates to a method for inhibiting cell proliferation, comprising contacting a cell with the compound of the present invention.

In another aspect the invention relates to the compound of the present invention for the preparation of a medicament for the treatment of a proliferative disorder or disease.

In another aspect the invention relates to a pharmaceutical composition comprising the compound of of the present invention and a pharmaceutically acceptable carrier, diluent or excipient, for the treatment of a proliferative disorder or disease.

In certain embodiments of a pharmaceutical package, method, use or pharmaceutical composition of the present invention, the proliferative disorder or disease is a cancer.

The invention further relates to a method of synthesizing the compound according to the present invention, comprising the step of reacting a compound having a structure represented by formula (II) with 2,4-dichloro-5-nitro-pyrimidine 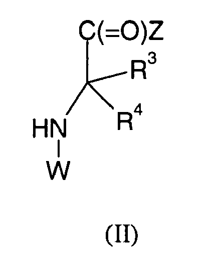 wherein W is-NH-P, wherein P is a protecting group, particularly: tert-butyloxycarbonyl (Boc), wherein Z is a leaving group, particularly -O-Me or O-Et, most particularly -O-Me, wherein R³ is -H and R⁴ is ethyl. In particular embodiments, the chiral carbon atom carrying substituents R³ and R⁴ has (R)-configuration.

In particular embodiments, W is -NH-Boc.

Furthermore, the invention relates to the compound having a structure represented by formula (III) 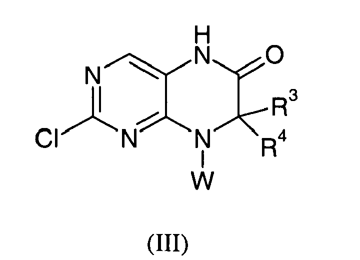 or any tautomeric or stereoisomeric form thereof, wherein R³, R⁴, and W are as defined above in this Section.

In particular embodiments, W is -NH-Boc. In particular embodiments, the chiral carbon atom in position 7 carrying substituents R³ and R⁴ has (R)-configuration.

### Examples

The compound of the present invention is shown in Table 1, with representative data characterizing certain of its physicochemical properties being shown in Table 2 (shown for racemate prior to enantiomeric resolution according to Example 2). The compound in Table 1 is synthesized according to Examples 1 and 2 below, or, alternatively, according to Example 1 when starting from the corresponding enantiomeric starting material, and the surprising inhibitory activities in biochemical assays, and anti-proliferative activities in cellular assays of this compound are shown in Table 3 (data shown for racemate prior to enantiomeric resolution according to Example 2), as determined according to Examples 3 and 4.

### A: Synthesis of 6-oxo-6,7-dihydro-5H-pteridin-8-yl derivatives (examples 1-2)

The compound of the invention may be prepared by the synthetic sequence shown in Scheme 1, and as shown in Examples 1 and 2. Alternatively, instead of obtaining compound 1 by enantiomeric resolution of the racemic mixture of compound 1 and the corresponding (S)-enantiomer, the pure (R)-enantiomer was directly obtained by starting from chiral (2S)-methyl-2-bromo-butyrate (or a corresponding compound carrying a sulfonyl-based leaving group instead of the bromine atom) instead of racemic methyl-2-bromo-butyrate in the synthesis of 2-(N'-tert-butoxycarbonyl-hydrazino)-butyric acid methyl ester according to example 1. A skilled artisan will appreciate that other routes of synthesis may be employed as well. In particular, other routes of synthesis may in fact be applied to certain aspects of the present invention. The skilled artisan is referred to general textbooks, such as March's Advanced Organic Chemistry (Michael B. Smith & Jerry March, Wiley-Interscience, 2000), The Practice of Medicinal Chemistry (Camile G. Wermuth, Academia Press, 2003) and Protective Groups in Organic Synthesis (Theosora W. Greene & Peter G.M. Wuts; John Wiley & Sons Inc, 1999).

Starting materials were commercially available or have been synthesized according to the following procedures:
(Trans)-4-(4-(cyclopropylmethyl)piperazin-1-yl)cyclohexanamine, 3HCl was prepared according to WO2007/090844

### Physicochemical data of racemic compound containing compound 1 prior to enantiomeric resolution (see Table 2):

The mass spectra (MS) data was generated on a LCMS system.

### Method A:

HPLC component: Agilent 1200 system, Waters Xterra column 5x50 mm (3.5 µm C18), 35°C, 0.1% formic acid aq. (SolvA) MeCN+0.1% formic acid v/v (SolvB), gradient 3%SolvB for 1.5 min, 3-70% SolvB 7 min; DAD 190-900 nm.

MS component: Waters ZQ. 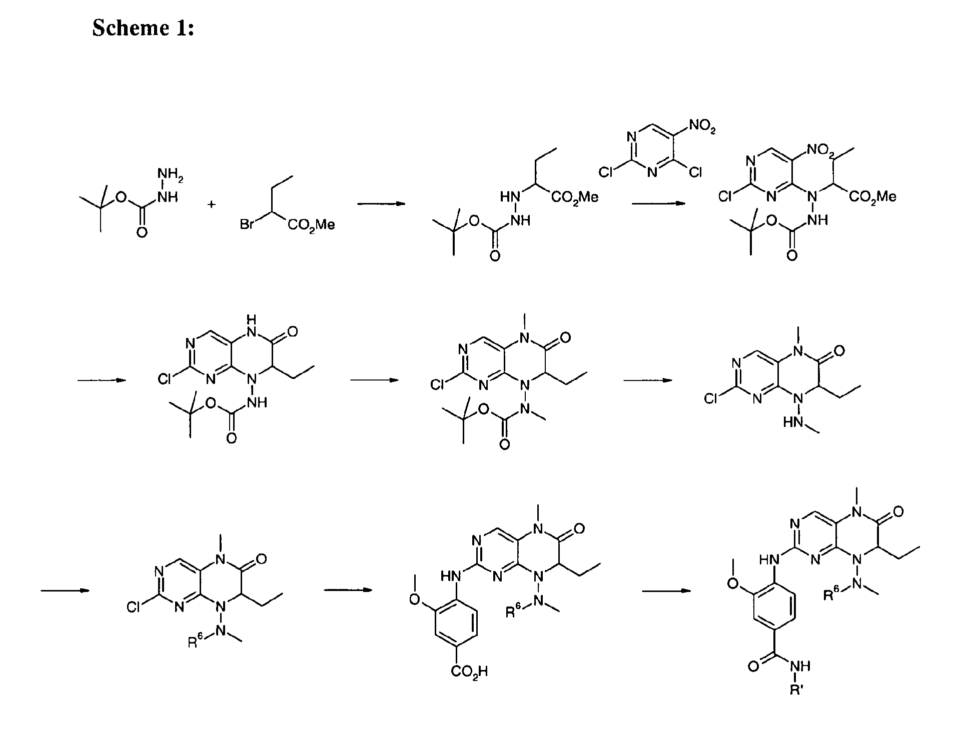 wherein R¹ is (trans)-4-(4-(cyclopropylmethyl)piperazin-1-yl)cyclohexyl, and R⁶ is - methyl.

### Example 1

### 2-(N'-tert-Butoxycarbonyl-hydrazino)-butyric acid methyl ester

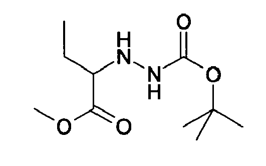

tert-Butyl carbazate (26.43 g; 0.2 mol) and DIPEA (28.43 g; 0.22 mol) were suspended in toluene (50 ml). Methyl-2-bromo-butyrate (36.21 g; 0.2 mol) was added dropwise at RT. After addition was complete, the mixture was heated under reflux for 7 h and left at RT for 3d. The mixture was diluted with Et₂O (250 ml) and stirred for 30 min. The precipitate was removed by filtration and washed with Et₂O (2 x 50 ml). The combined filtrate and washings were evaporated to dryness under reduced pressure and the residue was purified by flash chromatography (EtOAc/c-Hexane 1:3) to give 25.9 g (0.112 mol; 56%) of the title compound. Thin Layer Chromatography (TLC) (EtOAc/Hexanes 1:1): Rf = 0.50. MH⁺ = 233.

### 2-[N'-tert-Butoxycarbonyl-N-(2-chloro-5-nitro-pyrimidin-4-yl)-hydrazino]-butyric acid methyl ester

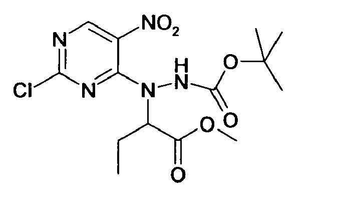

2-(N'-tert-Butoxycarbonyl-hydrazino)-butyric acid methyl ester (22.21g, 95.63 mmol) and K₂CO₃ (13.82 g; 100 mmol) was suspended in acetone (200 ml). Then 2,4-dichloro-5-nitro-pyrimidine (18.55 g; 95.63 mmol) was added dropwise at -35°C. After addition the reaction mixture was allowed to reach RT and stirring was continued for 20h. Then water (150 ml) was added and the mixture was extracted with EtOAc (3 x 150 ml). The combined organic extracts were washed with brine and dried over Na₂SO4. The solvent was removed under reduced pressure and the residue was purified by flash chromatography (EtOAc/c-Hexane 1:4) to give 35.9 g (92.1 mmol; 96%) of the title compound. TLC (EtOAc/Hexanes 1:4) Rf = 0.44. MH⁺ = 390; 392 (chlorine pattern)

### (2-Chloro-7-ethyl-6-oxo-6,7-dihydro-5H-pteridin-8-yl)-carbamic acid tert-butyl ester

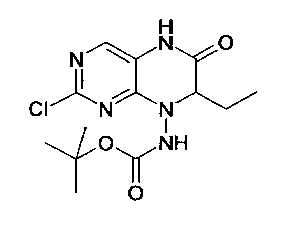

2-[N'-tert-Butoxycarbonyl-N-(2-chloro-5-nitro-pyrimidin-4-yl)-hydrazino]-butyric acid methyl ester (7218 mg; 18.5 mmol) was dissolved in EtOH (160 ml) and heated to 75°C. NH₄Cl (495 mg, 9.25 mmol) in water (100 ml) was added. Then Fe-powder (5171 mg, 92.6 mmol) was added portionwise. The mixture was stirred at 75°C for 20 min. The hot suspension was filtered through Celite. The red residue was washed with EtOH (4 x). The combined filtrate and washings were concentrated under reduced pressure to remove most of the EtOH. The aqueous suspension was extracted with EtOAc (1 x 320 ml), the organic extract was dried over Na₂SO₄ and filtered over Celite. The solvent was removed under reduced pressure to leave 30 g. Et₂O (50 ml) were added and the mixture was turned for 30 min. The precipitate was collected by filtration and washed with Et₂O to give 2730 mg of the title compound. The filtrate was evaporated to dryness and the residue was titurated with Et₂O (40 ml). The precipitate was collected by filtration and washed with Et₂O to give another 1g of the title compound. Yield: 3730 mg (11.4 mmol; 62%). TLC (EtOAc/Hexanes 1:1): Rf = 0.42. MH⁺ = 328; 330

### (2-Chloro-7-ethyl-5-methyl-6-oxo-6,7-dihydro-5H-pteridin-8-yl)-methyl-carbamic acid tert-butyl ester

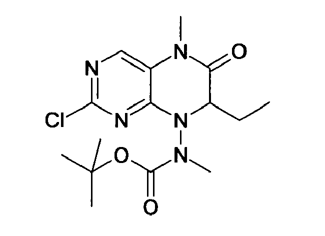

(2-Chloro-7-ethyl-6-oxo-6,7-dihydro-5H-pteridin-8-yl)-carbamic acid tert-butyl ester (2106 mg; 6.42 mmol) and K₂CO₃ (2391 mg ; 17.3 mmol) were suspended in DMF (12 ml) at RT. Methyl iodide (2100 mg; 14.8 mmol) were added dropwise. The mixture was stirred at RT for 5h. Residual methyl iodide was removed under reduced pressure and the mixture was poured into water (120 ml). The mixture was stirred and then centrifuged and the water was discarded. The residual oil was dissolved in dichloromethane (150 ml). The solution was washed with brine (100 ml) and dried over Na₂SO₄ After filtration the solvent was removed under reduced pressure to give 2158 mg (6.06 mmol, 94 %) of the title compound. TLC (EtOAc/Hexanes 1:1) Rf= 0.23, 0.39 (two spots). MH⁺ = 356; 358

### 2-Chloro-7-ethyl-5-methyl-8-methylamino-7,8-dihydro-5H-pteridin-6-one

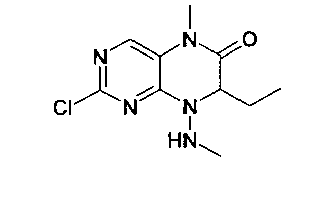

HCl was bubbled through i-PrOH (150 ml) for 45 min under ice-cooling. Then (2-chloro-7-ethyl-5-methyl-6-oxo-6,7-dihydro-5H-pteridin-8-yl)-methyl-carbamic acid tert-butyl ester (10.28 g; 28.9 mmol) was added and the mixture was stirred at 66°C for 1h. After cooling to RT the resulting precipitate was collected by filtration, washed with Et2O and dried in vacuo to give 8.0 g (24.3 mmol; 84%) white solid. To isolate the free base, the solid was suspended in dichloromethane (500 ml) and washed with sat. NaHCO₃-solution (150 ml). The organic layer was dried over Na₂SO₄, filtered and the solvent was removed under reduced pressure to give 6.22 g (24.3 mmol; 84%) of the title compound. TLC (EtOAc/Hexanes 1:1): Rf = 0.22. MH⁺ = 256; 258.

### 2-Chloro-8-dimethylamino-7-ethyl-5-methyl-7,8-dihydro-5H-pteridin-6-one

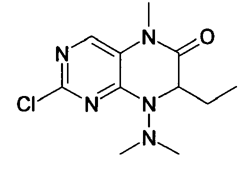

2-Chloro-7-ethyl-5-methyl-8-methylamino-7,8-dihydro-5H-pteridin-6-one (1195 mg; 4.67 mmol), formaldehyde (1.9 ml 37% aq. solution; 11.7 mmol) and HOAc (392 mg; 6.5 mmol) were dissolved in acetonitrile (20 ml) and stirred for 45 min. Sodium cyanoborohydride (890 mg; 14.2 mmol) in acetonitrile (20 ml) was added dropwise under cooling. The mixture was stirred for 15h at RT. Then formaldehyde (1.0 ml 37% aq. solution; 6.2 mmol), and HOAc (200 mg; 3.3 mmol) were added and the mixture was stirred for 1h. Sodium cyanoborohydride (480 mg; 7.6 mmol) was added and stirring was continued for 1h. The mixture was diluted with EtOAc (100 ml) and washed with NaHCO₃-solution (50 ml). The aqueous layer was back-extracted with EtOAc (1 x 30 ml), the combined organic layers were washed with brine (2 x 20 ml), dried over Na₂SO₄, filtered and evaporated to dryness. The residue was purified by flash chromatography to give 605 mg (2.24 mmol; 48%) of the title compound. TLC (2 x EtOAc/Hexanes 1:1): Rf = 0.5. MH⁺ = 270; 272.

### 4-(8-Dimethylamino-7-ethyl-5-methyl-6-oxo-5,6,7,8-tetrahydro-pteridin-2-ylamino)-3-methoxy-benzoic acid

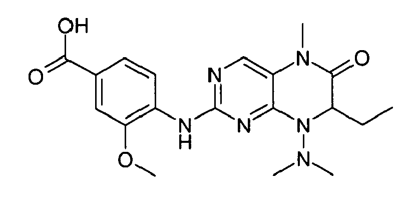

To a white suspension of 2-chloro-8-dimethylamino-7-ethyl-5-methyl-7,8-dihydro-5H-pteridin-6-one (1460 mg, 5.41 mmol) in acetonitrile (25 ml) was added 4-amino-3-methoxybenzoic acid (905 mg, 5.41 mmol) at RT followed by 2M aq. HCl (5.4 ml, 10.8 mmol). The mixture was heated under reflux (oil bath temp 90°C) for 35 h. The mixture was cooled to RT. The precipitate was collected by filtration, washed with MeOH (5 ml) and Et2O (5 ml) to give 885 mg of the title compound. The filtrate was heated under reflux for another 3 d and treated as above to give another 335 mg of the title compound. Total yield: 1220 mg (3.05 mmol, 56%). TLC (EtOH : Toluene : NH₃conc = 4:5: 1): Rf = 0.32. MH⁺ = 401.

### N-[trans-4-(4-Cyclopropylmethyl-piperazin-1-yl)-cyclohexyl]-4-(8-dimethylamino-7-ethyl-5-methyl-6-oxo-5,6,7,8-tetrahydro-pteridin-2-ylamino)-3-methoxy-benzamide (racemate including compound 1 and corresponding (S)-enantiomer)

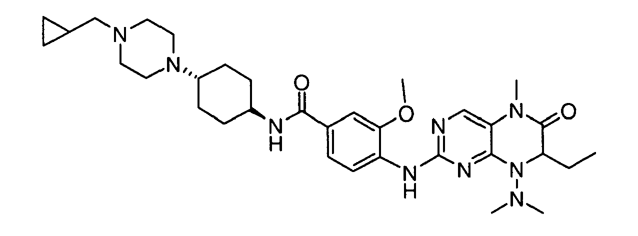

To a white suspension of 4-(8-dimethylamino-7-ethyl-5-methyl-6-oxo-5,6,7,8-tetrahydro-pteridin-2-ylamino)-3-methoxy-benzoic acid (86 mg, 0.215 mmol) in dichloromethane (15 ml) DIPEA (0.030 mg, 0.235 mmol) is added at RT followed by TBTU (72 mg, 0.225 mmol). The mixture is stirred at RT for 30 min. Then (trans)-4-(4-(cyclopropylmethyl)piperazin-1-yl)cyclohexanamine (54 mg, 0.228 mmol) in dichloromethane (2 ml) is added dropwise and the mixture is stirred at RT for 3d. The mixture is washed with water (1 x 1 ml), sat. NaHCO₃-solution (6 x 10 ml) and brine (1 x 10 ml). The organic solution is dried over Na₂SO₄, filtered and the solvent is removed under reduced pressure. The residue is purified by flash chromatography (dichloromethane / MeOH, 6:1 -> 5:1) to give the title compound. MH⁺ = 620.

### Example 2

### Enantiomeric separation of the racemate resulting from Example 1 by preparative HPLC

About 115 mg of the racemic compound resulting from Example 1 are separated into the pure enantiomers by preparative HPLC.

Preparative method:
Column: CHIRALPAK® IC 5 µm - 250 x 50 mm
Mobile phase: n-heptane / ethanol / diethylamine 70/30/0.1 (v/v/v)
Flow rate: 100 ml/min
Detection: UV 340 nm
Temperature: 25°C

Analytical method:
Column: CHIRALPAK® IC 5 µm - 250 x 4.6 mm
Mobile phase: n-heptane / ethanol / diethylamine 70/30/0.1 (v/v/v)
Flow rate: 1 ml/min
Detection: UV 340 nm
Temperature: 30°C

### Results:

The two enantiomers (compound 1 and the (S)-enantiomer) are obtained in amounts of more than about 40 mg each. The chemical purity (measured as area% at 340 nm) and the enantiomeric excess is greater than 95%.

### B: Biological Activity Assays (examples 3 to 5)

The biological activity and utility of the compound of the invention are demonstrated by one or more assays including those described in more detail below.

### Example 3: Biochemical assays for inhibition of kinase activity

### a) Plk1 kinase assay

| Kinase: PLK1 fl-His (Invitrogen; Cat.No. PV4038) | |
|---|---|
| IMAP Assay: | |
| Reaction Volume: | 8 µl |
| Reaction Time: | 60 min |
| Reaction Temperature: | room temperature |
| IMAP Incubation Time: | 60 min |
| Assay Plate: | 384 well U bottom, PP, black, low volume (Coming, 3676) |
| Compound Plate: | 384 well U bottom, PS (Falcon, 3995) |
| IMAP Binding Buffer A: | Molecular Devices, R7282 |
| IMAP Binding Buffer B: | Molecular Devices, R7283 |
| IMAP Binding Reagent: | Molecular Devices, R7207 |
| Controls: | |
| Negative Control (C-): | no kinase, no inhibitor |
| Positive Control (C+): | no inhibitor |
| Reaction buffer: | 20 mM Tris, pH 7.0 |
| | 1 mM DTT |
| | 2 mM MgCl₂ |
| | 2 mM MnCl₂ |
| | 0.01% Tween20 |
| Final Assay Concentrations: | |
| Kinase: | Kinase conc. yielding 50% substrate turnover as |
| | determined in titration experiment |
| | PLK1 fl-His (Invitrogen; PV4038) |
| ATP: | 0.63 µM |
| Substrate: 5FI-RGSFNDTLDFD-NH2 | 400 nM (jpt Peptide Technologies GmbH, |
| | Berlin, Germany) |
| IMAP Binding Solution: | 40 % IMAP Binding Buffer A |
| | 60 % IMAP Binding Buffer B |
| | IMAP Binding Reagent 1:1200 |

### Pipetting Sequence:

1) Add 6 µl 1.33-fold concentrated substrate + 1.33-fold concentrated ATP in 1-fold concentrated reaction buffer to each well of assay plate
2) Add 10.8 nl 740-fold concentrated inhibitor in 100% DMSO to each well except to C-and C+ wells using pintool (CyBio, Jena, Germany) (starting point: final inhibitor concentration 10 µM; IC₅₀ determination based on dilution series)
3) Add 10.8 nl 100% DMSO to C- and C+ wells using pintool
4) Add 2 µl reaction buffer to C- wells
5) Add 2 µl 4-fold concentrated kinase in reaction buffer to each well except C- wells
6) Incubate according to reaction time at room temperature
7) Add 15 µl IMAP binding solution to each well
8) Incubate according to IMAP incubation time at room temperature
9) Measure fluorescence polarization using integration time of 0.1 s (Analyst GT, Molecular Devices)

### b) Selectivity Panel:

The compound of the present invention was tested in a standard selectivity panel including Plkl and 255 other kinases (ProQinase GmbH, Freiburg, Germany), under conditions of non-disclosure, non-dissemination and non-use.

In brief, compound 1 (see Table 1) was tested at 1 µM in duplicates against Plk1 as well as against 255 other protein kinases, including Abl, Aktl, Aur A, Kit, Raf-1, Cdkl/CycB1, Chk1, FGF-R3, Flt3, Fyn, IGF1-R, INS-R, Lyn, p38a, PAK4, PKCalpha, Ret, ROCK2, Src, TIE2, and VEGF-R2. The test compound 1 inhibited Plk1 by more than about 90%, only three other kinases were inhibited by between about 50% and about 90%, about 13 kinases by between about 10% and about 50%, and all other kinases were inhibited by less than about 10%.

### Example 4: In vitro kinase activity of the compound of the present invention in cancer cell line

We observed the surprising fmding that the compound of the invention was able to penetrate cell membranes and retained their activity as Plk1 inhibitors against native Plk1 expressed within living cancer cells.

Cellular Plk1 kinase activity was measured by quantification of nucleophosmin (B23) Ser4 phosphorylation in a cancer cell line. Nucleophosmin (B23) Ser4 is phosphorylated by Plkl, and this phosphorylation can be specifically detected by cross-reactivity of a phospho-Mekl/2 antibody that is able to recognizes a mitotic-specific phosphorylated form of nucleophosmin (B23) in a nocodazole dependent way (Zhang, H., Shi, X., Paddon, H., Hampong, M., Dai, W., and Pelech, S. (2004) B23/Nuleophosmin Serin 4 Phosphorylation Mediates Mitotic Functions of Polo-like Kinase 1. J. Biol. Chem., 279(34), 35726-35734).

Briefly, HCT116 cells (ATCC, Order No. CCL-247) were plated in 96 well dishes 24 hours prior to nocodazole treatment at 25 000 cells per well. Cells were then treated with 100 ng/ml nocodazole (Sigma-Aldrich, # M1404). 20 hours after nocodazole treatment, cells were exposed to the test compound at various concentrations appropriate to determine an IC₅₀. 4 hours after compound addition, medium was carefully removed and 120 µl of Complete Tris Lysis Buffer (R60TX; MSD, Gaithersburg MD) was added to each well. Samples were then stored at - 20°C.

The level of nucleophosmin (B23) Ser4 phosphorylation in these cell lysis samples was estimated by electrochemiluminescence detection using the Meso Scale Discovery ("MSD") Technology Platform (MSD, Gaithersburg MD). The protocol used was as recommended by the manufacturer with the use of a phospho-Mekl/2 antibody (Cell Signalling, #9121) as primary antibody, and is as follows.

The cell lysates were thawed at room temperature and 40 µl were incubated on 96-well MULTI-ARRAY High Bind plates (L11XB; MSD) for 1.5 - 2 hours at room temperature. The supernatants were then removed and 150 µl of a 5% (w/v) solution of Blocker A (R93AA; MSD) in Tris Wash Buffer (R61TX; MSD) were added to each well. The plates were then incubated for 1 hour at room temperature before removal of the Blocker A solution and washed 4 times with 200 µl Tris Wash Buffer. 25 µl primary antibody (anti-phospho-MEK1/2, Cell Signalling, #9121, rabbit polyclonal), diluted 1:250 in a 1% (w/v) solution of Blocker A in Tris Wash Buffer, were added to each well. The plates were then incubated for 1 hour at room temperature before removal of the antibody solution and washed 4 times with 200 µl Tris Wash Buffer. Then, 25 µl of labelled secondary antibody (SULFO-TAG Goat Anti-Rabbit IgGAntibody (R32AB; MSD), diluted 1:500 in a 1% (w/v) solution of Blocker A in Tris Wash Buffer) were added to each well. The plates were then incubated for 1 hour at room temperature before removal of the antibody solution and washed 4 times with 200 µl Tris Wash Buffer. Then, 150 µl of 1x Read Buffer T (R92TC; MSD) were carefully added to each well and the plates were quantitatively detected for each well with a SECTOR™ Imager 6000 (MSD).

Inhibitory activity of the compound (IC) was calculated as % inhibition of nucleophosmin (B23) Ser4 phosphorylation compared to cells not treated with nocodazole. Table 3 represents the IC₅₀ values for inhibition of phosphorylation of nucleophosmin (B23) Ser4 for the compound of the invention showing that the compound demonstrated a clear ability to inhibit such phosphorylation, and hence act as inhibitors of native Plkl in a cancer cell line.

### Example 5: In vitro anti-proliferative activity of the compound of the present invention (in the racemate) against cancer cell lines

We observed the surprising finding that the compound of the invention in the racemate was useful in inhibiting proliferation of tumor cell lines (see Table 3).

**Table 7.1: Cell Lines used for demonstration of anti-proliferative activity**

| ATCC Name | ATCC Order No. | Doubling Time Td [hours] | Cells / well |
|---|---|---|---|
| HCT 116 | CCL-247 | 15 | 3500 |
| HT-29 | HTB-38 | 20 | 9000 |
| IMR-90 | CCL-186 | 53 | 8000 |

Cells were exposed to the test compound racemate at various concentrations appropriate to determine an IC₅₀ for 72 hours. Cell proliferation was measured using the SRB assay according to Shekan et al (J Natl Cancer Inst (1990) 82, 1107-112) in order to estimate the IC₅₀ values shown in Table 3. Briefly, cells were plated in 96 well dishes 24 hours prior to compound addition at the densities specified in Table 5.1. The assay was terminated with the addition of cold TCA to a final concentration of 10% after growth medium has been removed and the plates were incubated over night at 4°C. The plates were then washed 5 times with water and 100µl of a Sulforhodamine B solution (4%) was added to each well. The plates were then incubated for 10 minutes at room temperature before removal of unbound dye by washing with 1% acetic acid. The bound dye was solubilised with 10 mM Trizma base and the absorbance read at OD520.

Inhibitory activity of the compound (IC) was calculated as % inhibition of cell proliferation compared to cells treated with the solvent (DMSO). Table 3 represents the IC₅₀ values for inhibition of cell proliferation for the compound of the invention showing that the compound demonstrated a clear and pronounced anti-proliferative activity towards rapidly cycling cancer cell lines (HCT 116 and HT-29) compared to the slow cycling cell line (IMR90).

### Example 6: In vitro anti-proliferative activity against cell-lines representing a variety of cancer types

We observed that the compound of the invention (as racemic mixture containing compound 1 prior to enantiomeric resolution) was surprisingly able to inhibit the proliferation of a variety of tumour cell-lines representing different types of cancers.

IC₅₀ estimates for BI 2536 and for compounds 1 (in a racemic mixture containing compound 1 prior to enantiomeric resolution) (see Table 1) of the invention for inhibition of proliferation of the cell-lines shown in Table 8.1 (representing the various cancer types listed therein) were determined using the method of the previous example, with the appropriate number of cells per well.

**Table 8.1: 18-Cell Line Panel**

| ATCC Name | Cancer origin | ATCC Order No. | Cells / well |
|---|---|---|---|
| A-431 | skin | CRL-1555 | 9000 |
| A549 | lung | CCL-185 | 2500 |
| ACHN | kidney | CRL-1611 | 6000 |
| DU 145 | prostate | HTB-81 | 6000 |
| MDA-MB-468 | breast | HTB-132 | 8000 |
| NCI-H460 | NSCLC | HTB-177 | 3000 |
| OVCAR-3 | ovary | HTB-161 | 8000 |
| PC-3 | prostate | CRL-1435 | 4000 |
| SW620 | colon | CCL-227 | 9000 |
| A-375 | melanoma | CRL-1619 | 3500 |
| HT-29 | colon | HTB-38 | 9000 |
| HCT 116 | colon | CCL-247 | 4000 |
| HeLa | cervix | CCL-2 | 9000 |
| IMR-90 | lung | CCL-186 | 8000 |
| MDA-MB-231 | breast | HTB-26 | 9000 |
| SK-MEL-28 | melanoma | HTB-72 | 9000 |
| SK-MEL-5 | melanoma | HTB-70 | 3500 |
| COLO 205 | colon | CCL-222 | 9000 |

Table 8.2 shows that the compound of the invention (in the racemic mixture containing compound 1 prior to enantiomeric resolution) is able to inhibit the proliferation of a broad range of tumour cell-lines that represent different types of cancers. Indeed, despite its structural difference, the compound of the invention is surprisingly able to inhibit the proliferation of as general a broad class of cell-lines as the compound BI 2536, the only Plk inhibitor to have undergone clinical testing to date.

**Table 8.2: Results from 18-Cell Line Panel**

| | IC₅₀ [µM] | |
|---|---|---|
| ATCC Name | Compound 1 | BI 2536 |
| A-431 | < 1 | < 1 |
| A549 | < 1 | <1 |
| ACHN | < 1 | <1 |
| DU 145 | < 1 | <1 |
| MDA-MB-468 | > 1 | *plateau at* ∼*50%* |
| NCI-H460 | <1 | <1 |
| OVCAR-3 | < 1 | < 1 |
| PC-3 | < 1 | < 1 |
| SW620 | < 1 | < 1 |
| A-375 | < 1 | < 1 |
| HT-29 | < 1 | < 1 |
| HCT 116 | < 1 | < 1 |
| HeLa | < 1 | < 1 |
| IMR-90 | > 1 | > 1 |
| MDA-MB-231 | < 1 | < 1 |
| SK-MEL-28 | > 1 | < 1 |
| SK-MEL-5 | < 1 | < 1 |
| COLO 205 | < 1 | < 1 |

In a similar experiment, the inhibitory activity of compound 1 was tested against a panel of 57 tumor cell lines (OncoLead GmbH&Co.KG, Martinsried, Germany) under conditions of non-disclosure, non-dissemination and non-use.

In about 49 cell lines, GI₅₀ values (i.e. the concentration of test compound that results in a 50% reduction of tumor cell growth) below 1 µM. In 18 cell lines, the GI50 value was between 1 µM and 0.1 µM, and in 31 cell lines, the GI50 value was below 0.1 µM.

### Example 7: Activity of compound 1 in xenograft tumor models

### a) Mouse xenograft model using human HT-29 cells

With this assay we demonstrate the activity of the compound of the present invention against tumor cells using an in-vivo xenograft model.

### METHODS:

### Compound preparation

Compound 1 of the present invention (see Table 1) is prepared for i.p. administration in a biocompatible vehicle (e.g. in 10% DMA/50% PEG300/water; or in 5% Cremophor EL/5% ethanol/90% saline). The preparation is freshly made and injection volumes are adjusted to body weight (resulting in compound dosages of, for example, between 40 and 80mg/kg mouse).

### Mice/Husbandry.

Mice are obtained from Charles River Laboratory (CRL), housed in static microisolators, and provided *ad libitum* with water and an irradiated standard rodent diet (Purina Pico-Lab Rodent Diet 20).

### Standard Protocol.

### Experiments in Athymic mice.

Athymic nu/nu female mice (6-8 weeks old) from CRL are allowed to acclimate for at least 4 days. Human HT-29 cells (ATCC) colon carcinoma cell line are cultured in McCoy's medium (ATCC) supplied with 10% FCS and 1% Pen/Strep. The 2nd passage of cells with approximately 80% confluence is used in the study. Briefly, on Day 0, mice are inoculated with 0.1 ml (total 5x10⁶ cells) of HT-29 cell suspension (50x10⁶ cells/ml in McCoy's medium without supplements mixed 1:1 with Matrigel) by a subcutaneous injection into the lower right flank under light anesthesia. When the average tumor weights reaches above 100mg, animals with an appropriate tumor size are selected and are randomly divided into treatment groups (10 animals each).

Tumor growth and body weight are monitored and recorded twice a week. Tumors are measured by determining the length and width of the tumor with a digital caliper. Tumor weight is estimated using the following formula: Tumor Weight (mg) = (w² x 1) / 2 where w = width and I = length in mm of the tumor.

Tumor Growth Inhibition (TGI) % is calculated as follows: %TGI = 100(1-T/C) where T is the mean tumor size of the compound treated group on a given day, and C is the mean tumor size of the vehicle control group on the same day.

Toxic deaths are defined as deaths caused by compound treatment and not by advanced disease state. A death is considered toxic if the animal dies within 1 week after the final compound treatment and the tumor size has not reached 1000 mg non-tumor related deaths after this point are recorded, but not considered toxic deaths.

One group is treated with vehicle only (e.g. 10%DMA/50% PEG300/40% ddwater, po), and three groups of mice per compound and treatment schedule are treated with compound 1 in increasing amounts (e.g. with 40, 60 and 80 mg/kg doses dosed for five or ten days).

Mice are sacrificed when their tumors reach the 1000 mm³ endpoint volume. Treatment efficacy is determined as Log Cell Kill (LCK). LCK is a calculation that determines the percentage of tumor cells that are presumably killed after the initiation of treatment and can be used as a quantitative measure of efficacy: LCK = (T-C) / (3.32)(Td) where T = is the mean time required for the treatment group of mice to reach 1000 mg in size, C = the mean time for the control group tumors to reach 1000 mg in size, Td = is the Tumor Doubling time estimated from the linear regression analysis from a semi-log growth plot of the control group tumors during exponential growth and 3.32 = the number of doublings required for a population to increase 1-log10 unit. Each LCK unit represents 1-log10 unit of cell killing (e.g., 1 LCK = 90% kill, 2 LCK = 99% kill, etc.).

### b) Mouse xenograft model using human NCI-H460 cells

In a model similar to the one described in a), mice were inoculated with NCI-H460 tumor cells, and treated (9 mice for each treatment group) with either vehicle only (NMP:PEG300), compound 1 (70 mg/kg once per week), compound 1 (35 mg/kg twice per week), BI 6727 (70 mg/kg once per week) and gemcitabine (25 mg/kg, once per week) (vivoPharm Europe Limited, Munich, Germany). At the end of day four after begin of treatment, the mean body weight for the compound 1 treatment groups had dropped from 20.5 g to 19.2 g (~5%; standard deviation 2.6 g), and from 20.1 g to 19.3 g (-4%; standard deviation 0.9 g), respectively, and one mouse in the high-dose treatment group had died. In contrast, in the BI 6727 treatment group, the mean body weight had dropped from 20.2 g to 16.2 g (-20%; standard deviation 1.5 g), five mice had died and the remaining mice had to be killed for ethical reasons.

In the groups treated with compound 1, a reduction of tumor growth could be observed in comparison to the groups treated with vehicle only or with gemcitabine.

### C: Selection and development of drug candidates

### Example 8

In order to assess the suitability of compound 1 for use in a therapeutic composition for the treatment of disorders and diseases, such as cancers, additional data are collected. Such data can include the *in vitro* inhibition of the target molecule, such as a kinase, as measured by IC₅₀, or inhibition of proliferation across a panel of tumor cell lines, and tumor growth inhibition or reduction data and survival data from *in vivo* animal models. Furthermore, such experiments may also include the elucidation and/or determination of the mechanism of action of the subject compound, the target or target profile of the subject compound, and other characteristics of the subject compound, such as the binding affinity of the compound to the target(s) or the binding site of the compound on the target(s) and pharmacokinetic properties. Such experiments may also include molecular modelling of the drug-target interaction and the identification of metabolites formed after administration. Further experiments may include, for example, therapeutic profiling and toxicology in animals, phase I clinical trials in humans and other clinical trails.

One skilled in the art readily appreciates that the present invention is well adapted to carry out the objects and obtain the ends and advantages mentioned, as well as those inherent therein. The methods and reagents described herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

Those skilled in the art will recognize, or be able to ascertain using no more than routine experimentation, many equivalents to the specific embodiments of the invention described herein. Such equivalents are intended to be encompassed by the following claims. Those skilled in the art will also recognize that all combinations of embodiments, combination of aspects or features of the claims described herein are within the scope of the invention.

**Table 1: Compound of the present invention**

| No. | | |
|---|---|---|
| 1 | 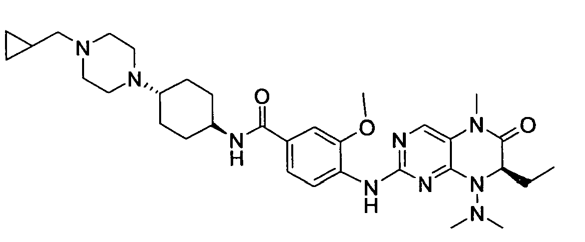 | (7R)-N-[trans-4-(4-Cyclopropylmethyl-piperazin-1-yl)-cyclohexyl]-4-(8-dimethylamino-7-ethyl-5-methyl-6-oxo-5,6,7,8-tetrahydro-pteridin-2-ylamino)-3-methoxy-benzamide |

**Table 2: Characterization of compound 1 (in racemic mixture)**

| Compound number | MH+ | Retention time | Method |
|---|---|---|---|
| racemic mixture containing compound 1 and (S)-enantiomer | 620 | 3.96 | A |

**Table 3: IC₅₀ values of compound 1 (in racemic mixture) in nucleophosmin assay (column 2); and inhibition of tumor cell growth (columns 3/5)**

| Compound number | Biochemical IC₅₀ [µM] Nucleophosmin assay | CELLULAR IC₅₀ [µM] | | |
|---|---|---|---|---|
| | | HCT 116 | HT-29 | IMR-90 |
| racemic mixture containing compound 1 and (S)-enantiomer | <0.1 | < 1 | < 1 | > 1 |

### Equivalents

Those skilled in the art will recognize, or be able to ascertain using no more than routine experimentation, many equivalents to the specific embodiments of the invention described herein. Such equivalents are intended to be encompassed by the following claims.

All of the above-cited references and publications are hereby incorporated by reference.

## Claims

1. The compound having the structure represented by formula (I) 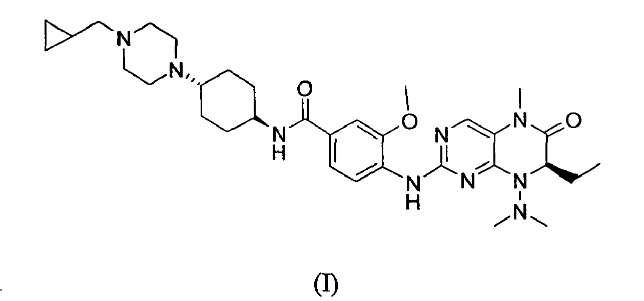 wherein the ring carbon atom in position 7 of the dihydropteridinone ring system has the (R)-configuration, or any tautomeric or stereoisomeric form thereof, provided that the stereoisomeric forms specifically designated in Formula (I), wherein the ring carbon atom in position 7 of the pteridinone ring system has the (R)-configuration, and the 1,4-substitution of the cyclohexane ring is in trans-configuration, are not changed and kept in the (R)- and trans-configuration, respectively ;
or any pharmaceutically acceptable salt or N-oxide thereof.

2. A pharmaceutical composition, including the compound of claim 1, and a pharmaceutically acceptable diluent, excipient or carrier.

3. The pharmaceutical composition of claim 2 for the treatment of a proliferative disease or disorder.

4. The pharmaceutical composition of claim 3, wherein said proliferative disease or disorder is cancer.
